# EUROPEAN PATENT APPLICATION

(11) **EP 4 846 839 A2**
(43) Date of publication of application: **02.12.2026**
(21) Application number: 26187916.7
(22) Date of filing: 09.07.2025
(51) Int. Cl.: A01J 7/04, A61N 5/06

(54) **UDDER IRRADIATION**

(30) Priority: 21.05.2025 US 202519214656
(62) Document number(s) of the earlier application(s) in accordance with Art. 76 EPC: 25188612.3 / 4 843 382
(71) Applicant: Hewitson, Andrew, Dana Point, California 92629 (US)
(72) Inventor: Hewitson, Andrew, Dana Point, California 92629 (US)
(74) Representative: Dickerson, David

(57) **Abstract**

A milking system, comprising a first milking cup structured to receive a first teat of an udder; a second milking cup structured to receive a second teat of the udder; and a laser irradiation device structured to bathe a surface of the udder in light at a wavelength greater than 400 nm and less than 1100 nm, wherein the light-bathed udder surface has an area of at least 100 cm².

## Description

### BACKGROUND OF THE DISCLOSURE

### FIELD OF THE DISCLOSURE

The present disclosure relates to an irradiation device as well as to a milking system and to related methods.

### DESCRIPTION OF THE RELATED ART

It is known to irradiate human tissue with infrared light for therapeutic reasons.

The present disclosure expounds upon this background.

### SUMMARY OF THE PRESENT DISCLOSURE

The aim of the present summary is to facilitate understanding of the present disclosure. The summary thus presents concepts and features of the present disclosure in a more simplified form and in looser terms than the detailed description below and should not be taken as limiting other portions of the present disclosure.

Loosely speaking, the present disclosure discloses an irradiation device having a plurality of laser diodes and/or light-emitting diodes configured to bathe an area of at least 100 cm² in near-infrared light, near-ultraviolet light, and/or light from the visible spectrum. For example, the light-emitting diodes may be distributed on a surface of at least 10 cm times 10 cm in area and may be configured to irradiate an area of at least 15 cm times 15 cm in an imaginary plane parallel to and 50 cm from the surface bearing the light-emitting diodes. The inventor has found that such a device is useful for irradiating an udder of a dairy animal such as a cow, goat, sheep, lama, or camel with positive effects on the quality of the milk.

Similarly, the present disclosure also discloses, *inter alia,* a milking system that, in addition to milking cups structured to receive teats of an udder, comprises an irradiation device structured to bathe an at least 100 cm² surface of the udder in near-infrared light, near-ultraviolet light, and/or light from the visible spectrum. The inventor has found that such a milking system is useful for irradiating an udder of a dairy animal such as a cow, goat, sheep, lama, or camel with positive effects on the quality of the milk.

Other objects, advantages and embodiments of the present disclosure will become apparent from the detailed description below, especially when considered in conjunction with the accompanying drawings.

### BRIEF DESCRIPTION OF THE DRAWINGS

The Figures show:
- Fig. 1A: a schematic depiction of an irradiation device in accordance with the present disclosure,
- Fig. 1B: a schematic depiction of a first cross-section of the irradiation device of Fig. 1A,
- Fig. 1C: a schematic depiction of a second cross-section of the irradiation device of Fig. 1A,
- Fig. 1D: a schematic depiction of a first cross-section of the irradiation device of Fig. 1C,
- Fig. 1E: a schematic depiction of a second cross-section of the irradiation device of Fig. 1C,
- Fig. 2: a schematic depiction of a milking system in accordance with the present disclosure,
- Fig. 3A: a schematic depiction of a first side of an irradiation device in accordance with the present disclosure,
- Fig. 3B: a schematic depiction of a second side of the irradiation device of Fig. 3A,
- Fig. 3C: a schematic depiction of a first cross-section of the irradiation device of Fig. 3A,
- Fig. 3D: a schematic depiction of a second cross-section of the irradiation device of Fig. 3A,
- Fig. 3E: a schematic depiction of the first side of the irradiation device of Fig. 3A,
- Fig. 3F: a schematic depiction of the second side of the irradiation device of Fig. 3A,
- Fig. 3G: a schematic depiction of a first cross-section of the irradiation device of Fig. 3E,
- Fig. 3H: a schematic depiction of a second cross-section of the irradiation device of Fig. 3E,
- Fig. 4A: a schematic depiction of a first side of an irradiation device in accordance with the present disclosure,
- Fig. 4B: a schematic depiction of a second side of the irradiation device of Fig. 4A,
- Fig. 4C: a schematic depiction of a cross-section of the irradiation device of Fig. 4A,
- Fig. 5A: a schematic depiction of a first side of an irradiation device in accordance with the present disclosure,
- Fig. 5B: a schematic depiction of a second side of the irradiation device of Fig. 5A,
- Fig. 5C: a schematic depiction of a cross-section of the irradiation device of Fig. 5A,
- Fig. 5D: a schematic depiction of a top side of the irradiation device of Fig. 5A,
- Fig. 6: a schematic depiction of a milking system in accordance with the present disclosure,
- Fig. 7: a schematic depiction of a milking system in accordance with the present disclosure,
- Fig. 8: a schematic depiction of a milking system in accordance with the present disclosure,
- Fig. 9: a first schematic depiction of a milking system in accordance with the present disclosure,
- Fig. 10A: a first schematic depiction of a milking system in accordance with the present disclosure,
- Fig. 10B: a second schematic depiction of the milking system of Fig. 10A,
- Figure 11A: a first schematic depiction of a milking system in accordance with the present disclosure,
- Figure 11B: a second schematic depiction of the milking system of Figure 11A,
- Figure 11C: a third schematic depiction of the milking system of Figure 11A,
- Figure 11D: a fourth schematic depiction of the milking system of Figure 10A,
- Figure 11E: a fifth schematic depiction of the milking system of Figure 11A,
- Figure 12A: a first schematic depiction of an irradiation system 1200 in accordance with the present disclosure,
- Figure 12B: a second schematic depiction of the irradiation system of Figure 12A,
- Figure 13: a first schematic depiction of an irradiation device in accordance with the present disclosure,
- Figure 14A: a first schematic depiction of an irradiation device in accordance with the present disclosure, and
- Figure 14B: a second schematic depiction of the irradiation device of Figure 14A.

### DETAILED DESCRIPTION

The various embodiments of the present disclosure and of the claimed invention, in terms of both structure and operation, will be best understood from the following detailed description, especially when considered in conjunction with the accompanying drawings.

Before elucidating the embodiments shown in the Figures, the various embodiments of the present disclosure will first be described in general terms.

The present disclosure discloses an irradiation device, *e.g.* a laser irradiation device. The irradiation device may be structured to bathe a surface of an udder in light at a wavelength greater than 400 nm and less than 1100 nm. The light-bathed udder surface may have an area of least 100 cm², at least 150 cm², at least 200 cm², at least 300 cm², at least 400 cm², or at least 600 cm².

The irradiation device may comprise a first plurality of semiconductor light emitters, *e.g.* a first plurality of light-emitting diodes and/or laser diodes. *(Hereinafter, the expression "SLE's" will be used as a shorthand notation for the plural term "semiconductor light emitters" and the expression "SLE" will be used as a shorthand notation for the singular term "semiconductor light emitter".)* The first plurality of SLE's may be a set of at least 5, at least 10, at least 15, or at least 20 SLE's. The first plurality of SLE's may be a set of not more than 40, not more than 30, or not more than 20 SLE's. Each SLE of the first plurality of SLE's may emit light at a wavelength greater than 800 nm and less than 1100 nm. Each SLE of the first plurality of SLE's may be a vertical-cavity surface-emitting laser (VCSEL).

The irradiation device may comprise a support. The support may be a unitary element or may comprise a plurality of components. For example, the support may comprise at least one component selected from the group consisting of a frame, a main body, a printed circuit board. The frame may be a multipart frame or unitary frame. The main body may be a multipart body or a unitary body. The support may comprise a first surface. For example, the support may comprise a (first) printed circuit board comprising a first surface. Similarly, the first surface may be a (first) surface of the main body. The first plurality of SLE's may be distributed on the first surface. In the present disclosure, the expression "SLE's ... distributed on ... a surface" may be understood as meaning that a minimally-sized polygon enclosing the SLE's occupies at least 80% or at least 90% of an area of the surface. In the present disclosure, the expression "SLE's ... distributed on ... a surface" may be understood as meaning that the SLE's are evenly and/or uniformly arranged on the surface. The first plurality of SLE's may be arranged in a regular pattern or in a symmetrical pattern. For example, the SLE's may be arranged in concentric circles around a central SLE, each circle being equidistant from its neighboring circle(s) / the central SLE, and each SLE being equidistant from its two nearest neighbors in the respective circle. The distance between neighboring circles may be chosen such that a minimum distance between any SLE in one circle and a respective, most proximate SLE of a neighboring circle is at least 80% and not more than 120% of a minimum distance between two neighboring SLE's of the respective one circle. *(An elucidation of the term "any" is given in the closing paragraphs of this specification.)* The distance between neighboring circles may be chosen such that a minimum distance between any SLE in one circle and a respective, most proximate SLE of a neighboring circle is at least 90% and not more than 110% of a minimum distance between two neighboring SLE's of the respective one circle. Similarly, the SLE's may be arranged in lines, each line being equidistant from its neighboring line(s), and each SLE being equidistant from its nearest neighbor(s) in the respective line. The SLE's in any line may be offset from the SLE's in a neighboring line, *e.g.* by half the distance between neighboring SLE's in a line. The distance between neighboring lines may be chosen such that a minimum distance between any SLE in one line and a respective, most proximate SLE of a neighboring line is at least 80% and not more than 120% of a minimum distance between two neighboring SLE's of the respective one line. The distance between neighboring lines may be chosen such that a minimum distance between any SLE in one line and a respective, most proximate SLE of a neighboring line is at least 90% and not more than 110% of a minimum distance between two neighboring SLE's of the respective one line.

The first surface may be a planar surface. The first surface may be a curved surface. The first surface may fit between two parallel imaginary planes separated by less than 15 cm, less than 10 cm, or less than 5 cm. The first surface may be a rectangular surface, a hexagonal surface, a polygonal surface, an oval surface, or a circular surface. The first surface may be at least 100 cm², at least 150 cm², at least 200 cm², at least 300 cm², at least 400 cm², or at least 600 cm² in area. The first surface may be less than 1000 cm², less than 600 cm², or less than 400 cm² in area. The first surface may be at least 10 cm times 10 cm in area. The first surface may be at least 15 cm times 10 cm in area, at least 15 cm times 15 cm in area, at least 20 cm times 15 cm in area, at least 20 cm times 20 cm in area, at least 25 cm times 20 cm in area, or at least 30 cm times 20 cm in area. The first plurality of SLE's may irradiate an area in a first imaginary plane, which irradiated area is larger than the area of the first surface. The first plurality of SLE's may irradiate an area in the first imaginary plane, which irradiated area is not larger than 2.5 times the area of the first surface. The first plurality of SLE's may irradiate an area in the first imaginary plane, which irradiated area is not larger than 4 times the area of the first surface. The first plurality of SLE's may irradiate an area of at least 15 cm times 15 cm in the first imaginary plane. The first plurality of SLE's may irradiate an area of at least 30 cm times 30 cm in the first imaginary plane. The first plurality of SLE's may irradiate an area in the first imaginary plane, *e.g.* as described above, such that an average irradiation intensity (effected by the first plurality of SLE's) in the area is not less than 40% of a maximum irradiation intensity (effected by the first plurality of SLE's) in the area. The irradiation device and/or the first plurality of SLE's may be structured such that the first plurality of SLE's irradiate an area in a first imaginary plane as described above. The first imaginary plane may be parallel to the first surface, e.g. parallel to a major side of an imaginary, minimally-sized rectangular parallelepiped cuboid enclosing the first surface. The first imaginary plane may be 50 cm from the first surface. For example, a minimum distance between the first imaginary plane and the first surface may be 50 cm.

The irradiation device may comprise a second plurality of semiconductor light emitters, *e.g.* a second plurality of light-emitting diodes and/or laser diodes. The second plurality of SLE's may be a set of at least 5, at least 10, at least 15, or at least 20 SLE's. The second plurality of SLE's may be a set of not more than 40, not more than 30, or not more than 20 SLE's. Each SLE of the second plurality of SLE's may emit light at a wavelength greater than 400 nm and less than 800 nm. Each SLE of the second plurality of SLE's may emit light at a wavelength greater than 600 nm and less than 800 nm. The second plurality of SLE's may be distinct from the first plurality of SLE's.

The irradiation device may comprise a third plurality of semiconductor light emitters, e.g. a third plurality of light-emitting diodes and/or laser diodes. The third plurality of SLE's may be a set of at least 5, at least 10, at least 15, or at least 20 SLE's. The third plurality of SLE's may be a set of not more than 40, not more than 30, or not more than 20 SLE's. Each SLE of the third plurality of SLE's may emit light at a wavelength greater than 400 nm and less than 600 nm. The third plurality of SLE's may be distinct from second plurality of SLE's and the first plurality of SLE's.

The second and/or third plurality of SLE's may be distributed on the first surface. (Hereinafter, the expression "the additional SLE's" will be used as shorthand in lieu of the cumbersome expression "the second and/or third plurality of SLE's".) As such, the additional SLE's may be arranged on the first surface such that a minimally-sized polygon enclosing the additional SLE's occupies at least 80% or at least 90% of an area of the first surface. Similarly, the additional SLE's may be evenly and/or uniformly arranged on the first surface. The additional SLE's may be arranged in a regular pattern or in a symmetrical pattern, e.g. in a pattern of concentric circles or equidistant lines as detailed above.

The additional SLE's may irradiate an area in the first imaginary plane, which irradiated area is larger than the area of the first surface. The additional SLE's may irradiate an area in the first imaginary plane, which irradiated area is not larger than 2.5 times the area of the first surface. The additional SLE's may irradiate an area in the first imaginary plane, which irradiated area is not larger than 4 times the area of the first surface. The additional SLE's may irradiate an area of at least 15 cm times 15 cm in the first imaginary plane. The additional SLE's may irradiate an area of at least 30 cm times 30 cm in the first imaginary plane. The additional SLE's may irradiate an area in the first imaginary plane, *e.g.* as described above, such that an average irradiation intensity (effected by the additional SLE's) in the area is not less than 40% of a maximum irradiation intensity (effected by the additional SLE's) in the area. The irradiation device and/or the additional SLE's may be structured such that the additional SLE's irradiate an area in the first imaginary plane as described above.

The irradiation device may comprise a first plurality of lenses. The first plurality of lenses may focus light emitted by the first plurality of SLE's and/or the second plurality of SLE's and/or the third plurality of SLE's. Each individual lens of the first plurality of lenses may be structured to focus a cone of light diverging at an aperture angle of greater than 40°, greater than 50° or greater than 60° to a cone of light diverging at an aperture angle of less than 30°, less than 20° or less than 10°. For each individual SLE of the first plurality of SLE's, the first plurality of lenses may comprise a (single) corresponding lens. For each individual SLE of the second plurality of SLE's, the first plurality of lenses may comprise a (single) corresponding lens. For each individual SLE of the third plurality of SLE's, the first plurality of lenses may comprise a (single) corresponding lens. The first plurality of lenses may be distributed on a first lens carrier, *e.g.* such that a minimally-sized polygon enclosing the first plurality of lenses occupies at least 80% or at least 90% of an area of the first lens carrier and/or such that the lenses are evenly and/or uniformly arranged on the first lens carrier. The first plurality of lenses may be arranged such that, for each individual SLE of the first plurality of SLE's, a corresponding one lens of the first plurality of lenses is situated in a beam of light emitted by the respective individual SLE. The first plurality of lenses may be arranged such that, for each individual SLE of the first plurality of SLE's, at least 80% of a light emitted by the respective individual SLE passes through a corresponding one lens of the first plurality of lenses. The first plurality of lenses may be arranged such that, for each individual SLE of the second plurality of SLE's and/or the third plurality of SLE's, a corresponding one lens of the first plurality of lenses is situated in a beam of light emitted by the respective individual SLE. The first plurality of lenses may be arranged such that, for each individual SLE of the second plurality of SLE's and/or the third plurality of SLE's, at least 80% of a light emitted by the respective individual SLE passes through a corresponding one lens of the first plurality of lenses. A combination of an individual SLE and a corresponding lens may be termed a "focussed semiconductor light emitter" or "focussed SLE". A major surface of the first lens carrier may be parallel to the first surface. The first lens carrier may be structured and arranged such that a minimum distance from any of the first plurality of SLE's and/or the additional SLE's to the first lens carrier is not more than 10 mm, not more than 8 mm, not more 5 mm, or not more than 3 mm. The first lens carrier may consist of a polymer material. The first lens carrier may exhibit a transmissibility of at least 70% for light in the range of greater than 400 nm and less than 1100 nm. The first plurality of lenses may be integrally formed with first lens carrier.

At least one SLE of the first plurality of SLE's may emit light in a first direction. For example, each or any SLE of the first plurality of SL''s may emit light in the first direction. The first direction may be a direction relative to the irradiation device as a whole and/or a direction relative to the first surface. Each (focussed) SLE of the first plurality of SLE's may, *e.g.* in combination with a respective lens of the first plurality of lenses, emit light in a direction that does not differ from said first direction by more than 30°, by more than 20°, by more than 10°, or by more than 5°. At least one SLE of the additional SLE's may, *e.g.* in combination with a respective lens of the first plurality of lenses, emit light in the first direction. For example, each or any SLE of the additional SLE's may emit light in the first direction. Each (focussed) SLE of the additional SLE's may emit light in a direction that does not differ from said first direction by more than 30°, by more than 20°, by more than 10°, or by more than 5°.

The irradiation device may comprise a fourth plurality of semiconductor light emitters, *e.g.* a fourth plurality of light-emitting diodes and/or laser diodes. The fourth plurality of SLE's may be a set of at least 5, at least 10, at least 15, or at least 20 SLE's. The fourth plurality of SLE's may be a set of not more than 40, not more than 30, or not more than 20 SLE'S. Each SLE of the second plurality of SLE's may emit light at a wavelength greater than 800 nm and less than 1100 nm. Each SLE of the fourth plurality of SLE's may be a vertical-cavity surface-emitting laser (VCSEL).

The support may comprise a second surface. For example, the support may comprise a (second) printed circuit board comprising a second surface. Similarly, the second surface may be a (second) surface of the main body. The fourth plurality of SLE's may be distributed on the second surface. As such, the fourth plurality of SLE's may be arranged on the second surface such that a minimally-sized polygon enclosing the fourth plurality of SLE's occupies at least 80% or at least 90% of an area of the second surface. Similarly, the fourth plurality of SLE's may be evenly and/or uniformly arranged on the second surface. The fourth plurality of SLE's may be arranged in a regular pattern or in a symmetrical pattern, *e.g.* in a pattern of concentric circles or equidistant lines as detailed above.

The second surface may be a planar surface. The second surface may be a curved surface. The second surface may fit between two parallel imaginary planes separated by less than 15 cm, less than 10 cm, or less than 5 cm. The second surface may be a rectangular surface, a hexagonal surface, a polygonal surface, an oval surface, or a circular surface. The second**CLEAN COPY** surface may be at least 100 cm², 150 cm², 200 cm², 300 cm², 400 cm², or 600 cm² in area. The second surface may be less than 1000 cm², less than 600 cm², or less than 400 cm² in area. The second surface may be at least 10 cm times 10 cm in area. The second surface may be at least 15 cm times 10 cm in area, at least 15 cm times 15 cm in area, at least 20 cm times 15 cm in area, at least 20 cm times 20 cm in area, at least 25 cm times 20 cm in area, or at least 30 cm times 20 cm in area. The fourth plurality of SLE's may irradiate an area in a second imaginary plane, which irradiated area is larger than the area of the second surface. The fourth plurality of SLE's may irradiate an area in the second imaginary plane, which irradiated area is not larger than 2.5 times the area of the second surface. The fourth plurality of SLE's may irradiate an area in the second imaginary plane, which irradiated area is not larger than 4 times the area of the second surface. The fourth plurality of SLE's may irradiate an area of at least 15 cm times 15 cm in the second imaginary plane. The fourth plurality of SLE's may irradiate an area of at least 30 cm times 30 cm in the second imaginary plane. The fourth plurality of SLE's may irradiate an area in the second imaginary plane, *e.g.* as described above, such that an average irradiation intensity (effected by the fourth plurality of SLE's) in the area is not less than 40% of a maximum irradiation intensity (effected by the fourth plurality of SLE's) in the area. The irradiation device and/or the fourth plurality of SLE's may be structured such that the fourth plurality of SLE's irradiate an area in a second imaginary plane as described above. The second imaginary plane may be parallel to the second surface, *e.g.* parallel to a major side of an imaginary, minimally-sized rectangular parallelepiped cuboid enclosing the second surface. The second imaginary plane may be 50 cm from the second surface. For example, a minimum distance between the second imaginary plane and the second surface may be 50 cm.

The irradiation device may comprise a fifth plurality of semiconductor light emitters, *e.g.* a fifth plurality of light-emitting diodes and/or laser diodes. The fifth plurality of SLE's may be a set of at least 5, at least 10, at least 15, or at least 20 SLE's. The fifth plurality of SLE's may be a set of not more than 40, not more than 30, or not more than 20 SLE's. Each SLE of the fifth plurality of SLE's may emit light at a wavelength greater than 400 nm and less than 800 nm. Each SLE of the fifth plurality of SLE's may emit light at a wavelength greater than 600 nm and less than 800 nm. The fifth plurality of SLE's may be distinct from the first plurality of SLE's, the second plurality of SLE's, the third plurality of SLE's, and the fourth plurality of SLE's.

The irradiation device may comprise a sixth plurality of semiconductor light emitters, *e.g.* a sixth plurality of light-emitting diodes and/or laser diodes. The sixth plurality of SLE's may be a set of at least 5, at least 10, at least 15, or at least 20 SLE's. The sixth plurality of SLE's may be a set of not more than 40, not more than 30, or not more than 20 SLE's. Each SLE of the sixth plurality of SLE's may emit light at a wavelength greater than 400 nm and less than 600 nm. The sixth plurality of SLE's may be distinct from the first plurality of SLE's, the second plurality of SLE's, the third plurality of SLE's, the fourth plurality of SLE's, and the fifth plurality SLE's.

The fifth and/or sixth plurality of SLE's may be distributed on the second surface. (Hereinafter, the expression "the other additional SLE's" will be used as shorthand in lieu of the cumbersome expression "the fifth and/or sixth plurality of SLE's".) As such, the other additional SLE's may be arranged on the second surface such that a minimally-sized polygon enclosing the other additional SLE's occupies at least 80% or at least 90% of an area of the second surface. Similarly, the other additional SLE's may be evenly and/or uniformly arranged on the second surface. The other additional SLE's may be arranged in a regular pattern or in a symmetrical pattern, e.g. in a pattern of concentric circles or equidistant lines as detailed above.

The other additional SLE's may irradiate an area in the second imaginary plane, which irradiated area is larger than the area of the second surface. The other additional SLE's may irradiate an area in the second imaginary plane, which irradiated area is not larger than 2.5 times the area of the second surface. The other additional SLE's may irradiate an area in the second imaginary plane, which irradiated area is not larger than 4 times the area of the second surface. The other additional SLE's may irradiate an area of at least 15 cm times 15 cm in the second imaginary plane. The other additional SLE's may irradiate an area of at least 30 cm times 30 cm in the second imaginary plane. The other additional SLE's may irradiate an area in the second imaginary plane, *e.g.* as described above, such that an average irradiation intensity (effected by the other additional SLE's) in the area is not less than 40% of a maximum irradiation intensity (effected by the other additional SLE's) in the area. The irradiation device and/or the other additional SLE's may be structured such that the other additional SLE's irradiate an area in the second imaginary plane as described above.

The irradiation device may comprise a second plurality of lenses. The second plurality of lenses may focus light emitted by the fourth plurality of SLE's and/or the fifth plurality of SLE's and/or the sixth plurality of SLE's. For each individual SLE of the fourth plurality of SLE's, the second plurality of lenses may comprise a (single) corresponding lens. For each individual SLE of the fifth plurality of SLE's, the second plurality of lenses may comprise a (single) corresponding lens. For each individual SLE of the sixth plurality of SLE's, the second plurality of lenses may comprise a (single) corresponding lens. Each individual lens of the first plurality of lenses may be structured to focus a cone of light diverging at an aperture angle of greater than 40°, greater than 50° or greater than 60° to a cone of light diverging at an aperture angle of less than 30°, less than 20° or less than 10°. The second plurality of lenses may be distributed on a second lens carrier, *e.g.* such that a minimally-sized polygon enclosing the second plurality of lenses occupies at least 80% or at least 90% of an area of the second lens carrier and/or such that the lenses are evenly and/or uniformly arranged on the second lens carrier. The second plurality of lenses may be arranged such that, for each individual SLE of the fourth plurality of SLE's, a corresponding one lens of the second plurality of lenses is situated in a beam of light emitted by the respective individual SLE. The second plurality of lenses may be arranged such that, for each individual SLE of the fourth plurality of SLE's, at least 80% of a light emitted by the respective individual SLE passes through a corresponding one lens of the second plurality of lenses. The second plurality of lenses may be arranged such that, for each individual SLE of the fifth plurality of SLE's and/or the sixth plurality of SLE's, a corresponding one lens of the second plurality of lenses is situated in a beam of light emitted by the respective individual SLE. The second plurality of lenses may be arranged such that, for each individual SLE of the fifth plurality of SLE's and/or the sixth plurality of SLE's, at least 80% of a light emitted by the respective individual SLE passes through a corresponding one lens of the second plurality of lenses. As already stated above, combination of an individual SLE and a corresponding lens may be termed a "focussed semiconductor light emitter" or "focussed SLE". A major surface of the second lens carrier may be parallel to the second surface. The second lens carrier may be structured and arranged such that a minimum distance from any of the second plurality of SLE's and/or the other additional SLE's to the second lens carrier is not more than 10 mm, not more than 8 mm, not more 5 mm, or not more than 3 mm. The second lens carrier may consist of a polymer material. The second lens carrier may exhibit a transmissibility of at least 70% for light in the range of greater than 400 nm and less than 1100 nm. The second plurality of lenses may be integrally formed with second lens carrier.

At least one SLE of the fourth plurality of SLE's may emit light in a second direction. For example, each or any SLE of the fourth plurality of SLE's may emit light in the second direction. The second direction may be a direction relative to the irradiation device as a whole and/or a direction relative to the second surface. Each (focussed) SLE of the second plurality of SLE's may, *e.g.* in combination with a respective lens of the second plurality of lenses, emit light in a direction that does not differ from said second direction by more than 30°, by more than 20°, by more than 10°, or by more than 5°. At least one SLE of the other additional SLE's may emit light in the second direction. For example, each or any SLE of the other additional SLE's may emit light in the second direction. Each (focussed) SLE of the other additional SLE's may, *e.g.* in combination with a respective lens of the second plurality of lenses, emit light in a direction that does not differ from said second direction by more than 30°, by more than 20°, by more than 10°, or by more than 5°.

The first direction may be perpendicular to the first surface, *e.g.* perpendicular to a major side of an imaginary, minimally-sized rectangular parallelepiped cuboid enclosing the first surface. The second direction may be perpendicular to the second surface, *e.g.* perpendicular to a major side of an imaginary, minimally-sized rectangular parallelepiped cuboid enclosing the second surface. The irradiation device may be structured such that, in a standard operating state of the irradiation device, the first direction is horizontal or angled downward by not more than 20°, not more than 15°, not more than 10°, or not more than 5°. The irradiation device may be structured such that, in a standard operating state of the irradiation device, the second direction is horizontal or angled downward by not more than 20°, not more than 15°, not more than 10°, or not more than 5°. The first surface and the second surface may face in generally opposite directions. The first surface may be arranged in mirror-symmetric arrangement to the second surface. A plane of mirror symmetry defined by the first surface and the second surface may constitute a vertical plane of the irradiation device. In the standard operating state of the irradiation device, the vertical plane may be oriented vertically. The first surface may be parallel to the second surface. A major side of an imaginary, minimally-sized rectangular parallelepiped cuboid enclosing the first surface may be parallel to a major side of an imaginary, minimally-sized rectangular parallelepiped cuboid enclosing the second surface. Similarly, the first surface may be angled to the second surface, *e.g.* by less than 40°, less than 30°, less than 20°, or less than 10° (as measured in any of a vertical plane perpendicular to the first surface, a vertical plane perpendicular to the second surface, a vertical plane perpendicular to the plane of mirror symmetry, and a horizontal plane). An angle between a major side of an imaginary, minimally-sized rectangular parallelepiped cuboid enclosing the first surface and a major side of an imaginary, minimally-sized rectangular parallelepiped cuboid enclosing the second surface may be less than 40°, less than 30°, less than 20°, or less than 10°. For example, the first surface may be angled to the second surface as measured in a vertical plane while being parallel to the second surface as measured in a horizontal plane. The second direction may be antiparallel to the first direction. The second direction may be within 40°, within 30°, within 20°, or within 10° of opposite the first direction, *e.g.* as measured in any of a vertical plane perpendicular to the first surface, a vertical plane perpendicular to the second surface, a vertical plane perpendicular to the plane of mirror symmetry, and a horizontal plane.

The irradiation device may comprise a power supply system. The power supply system may supply electrical power to the first plurality of SLE's and/or the fourth plurality of SLE's and/or the additional SLE's and/or the other additional SLE's *(Hereinafter, the expression "the light emitters" will be used as shorthand in lieu of the cumbersome expression "the first plurality of SLE's and*/*or the additional SLE's and*/*or the fourth plurality of SLE's and*/*or the other additional SLE's".)*. The power supply system may comprise a voltage converter, *e.g.* a voltage converter that converts a line voltage of 110 V or 220 V to a voltage of less than 40 V, or less than 20 V. The power supply system may comprise a current modulator, *e.g.* a current modulator that modulates a current supplied to any of the light emitters. The current modulation may effect pulsation of light emitted by the respective light emitters. The frequency of the current modulation may be dependent on a wavelength of light emitted by the respective light emitters. The power supply system may comprise a plurality of wires and/or conductive traces. The plurality of wires and/or conductive traces may comprise wires and/or conductive traces that interconnect other elements of the power supply system. The plurality of wires and/or conductive traces may comprise wires and/or conductive traces that connect any of the individual SLE's with elements of the power supply system. The irradiation device may comprise a power cord, *e.g.* a power cord that connects the power supply system to a domestic power outlet. The power cord may extend from a main body of the irradiation device.

The irradiation device may comprise at least one detector, *e.g.* a detector structured to distinguish between a presence and an absence of an irradiation target and/or an udder detector structured to detect a location of an udder. The irradiation device may comprise a first detector structured to distinguish between a presence and an absence of an irradiation target, *e.g.* a dairy animal such as a cow, goat, sheep, lama, or camel, in an irradiation zone of the first plurality of SLE's. The irradiation zone of the first plurality of SLE's may be an imaginary zone extending not more than 1 m from the irradiation device in the first direction. The irradiation device may comprise a second detector structured to distinguish between a presence and an absence of an irradiation target, *e.g.* a dairy animal such as a cow, goat, sheep, lama, or camel, in an irradiation zone of the fourth plurality of SLE's. The irradiation zone of the fourth plurality of SLE's may be an imaginary zone extending not more than 1 m from the irradiation device in the second direction. The detector may comprise an infrared sensor. The detector may comprise a time-of-flight detector. The detector may comprise a camera. The detector may comprise a processor and an image processing software that, when executed on the processor, processes at least one image obtained from a camera to provide at least one of an output indicative of a presence of an irradiation target (in the at least one camera image) and an output indicative of an absence of an irradiation target (in the at least one camera image). The detector may comprise a processor and an image processing software that, when executed on the processor, processes at least one image obtained from a camera to provide at least one of an output indicative of a location of an udder (relative to the irradiation device). The detector may comprise a switch. The switch may be situated outside the main body of the irradiation device. The switch may be connected to the main body of the irradiation device by a cord. The switch may be structured such that the switch (arranged in a detection position) adopts a first operating state in the presence of an irradiation target. The switch may be structured such that the switch (arranged in a detection position) adopts a second operating state in the absence of an irradiation target. The switch may be a mechanic switch.

The irradiation device may comprise at least one controller. The controller may be structured to interrupt a supply of power to the first plurality of SLE's and/or the additional SLE's in response to a signal from the first detector indicative of an absence of an irradiation target. The controller may be structured to interrupt a supply of power to the fourth plurality of SLE's and/or the other additional SLE's in response to a signal from the second detector indicative of an absence of an irradiation target. The controller may be structured to incrementally increase a supply of power to the light emitters after an interruption of the supply of power. The controller may incrementally increase the supply of power at a rate slow enough that a nominal full power is not attainable until at least 200 milliseconds, at least 400 milliseconds, at least 500 milliseconds, at least 800 milliseconds, at least 1,000 milliseconds, at least 1,500 milliseconds, or at least 2,000 milliseconds have elapsed since reestablishment of power. The controller may incrementally increase the supply of power at a rate fast enough that a nominal full power is attainable within 5 seconds, within 3 seconds, within 2 seconds, or within one second of reestablishment of power. The controller may control the current modulation effected by the current modulator, *e.g.* as a function of a user input.

The controller may be structured to interrupt a supply of power to at least one subset of the first plurality of SLE's and/or the additional SLE's in response to a signal from a detector indicative of an absence of an udder in an irradiation zone of the at least one subset, e.g. in response to a signal from an udder detector indicative of an udder location outside an irradiation zone of the at least one subset. The controller may use signals obtained from a detector, *e.g.* an udder detector, to interrupt a supply of power to at least one subset of the first plurality of SLE's and/or the additional SLE's such that at least 80% of a light emitted by the first plurality of SLE's and/or the additional SLE's irradiate an udder. Similarly, the controller may be structured to interrupt a supply of power to another at least one subset of the fourth plurality of SLE's and/or the other additional SLE's in response to a signal from a detector indicative of an absence of an udder in an irradiation zone of the other at least one subset, *e.g.* in response to a signal from an udder detector indicative of an udder location outside an irradiation zone of the at least one subset. The controller may use signals obtained from a detector, *e.g.* an udder detector, to interrupt a supply of power to another at least one subset of the fourth plurality of SLE's and/or the other additional SLE's such that at least 80% of a light emitted by the fourth plurality of SLE's and/or the other additional SLE's irradiate an udder.

The irradiation device may comprise at least one timer. The timer may measure an elapsed time since receipt of a signal, *e.g.* from the first detector and/or the second detector, indicative of a presence of an irradiation target. The irradiation device may be structured to effect a first timing sequence, *e.g.* in response to a signal indicative of a presence of an irradiation target. The first timing sequence may comprise controlling operation of any of the light emitters as a function of time, *e.g.* as a function of a time measured by the timer. The first timing sequence may comprise interrupting a supply of power to any of the light emitters as a function of time, *e.g.* as a function of a time measured by the timer. The first timing sequence may comprise interrupting a supply of power to the first plurality of SLE's and/or the additional SLE's as a function of time, *e.g.* in response to a signal from the first detector indicative of a presence of an irradiation target. The first timing sequence may comprise interrupting a supply of power to the fourth plurality of SLE's and/or the other additional SLE's as a function of time, *e.g.* in response to a signal from the second detector indicative of a presence of an irradiation target. The first timing sequence may comprise interrupting a supply of power to the first plurality of SLE's and/or the additional SLE's for a first length of time measured from receipt of a signal from the first detector indicative of a presence of an irradiation target. The first timing sequence may comprise interrupting a supply of power to the fourth plurality of SLE's and/or the other additional SLE's for a second length of time measured from receipt of a signal from the second detector indicative of a presence of an irradiation target. The first timing sequence may comprise interrupting a supply of power to the first plurality of SLE's and/or the additional SLE's after elapse of a third length of time measured from receipt of a signal from the first detector indicative of a presence of an irradiation target. The first timing sequence may comprise interrupting a supply of power to the fourth plurality of SLE's and/or the other additional SLE's after elapse of a fourth length of time measured from receipt of a signal from the second detector indicative of a presence of an irradiation target. The first length of time and/or the second length of time may be longer than 20 seconds, longer than 30 seconds, or longer than 40 seconds. The first length of time and/or the second length of time may be shorter than 90 seconds, shorter than 80 seconds, shorter than 70 seconds, or shorter than 60 seconds. The third length of time and/or the fourth length of time may be longer than 60 seconds, longer than 90 seconds, longer than 120 seconds, longer than 180 seconds, or longer than 240 seconds. The third length of time and/or the fourth length of time may be shorter than 300 seconds, shorter than 240 seconds, shorter than 180 seconds, or shorter than 120 seconds.

The irradiation device may comprise at least one clock. The clock may establish a current time, *e.g.* current local time. The clock may establish a current date, *e.g.* current local date. The irradiation device may be structured to effect a second timing sequence. The second timing sequence may comprise controlling operation of any of the light emitters as a function of time, *e.g.* as a function of the current date and/or time established by the clock. The second timing sequence may comprise interrupting a supply of power to any of the light emitters as a function of time, *e.g.* as a function of the current date and/or time established by the clock. The second timing sequence may comprise interrupting a supply of power to all or a first subset of the light emitters during a first time period. The second timing sequence may comprise interrupting a supply of power to all or a second subset, distinct from the first subset, of the light emitters during a second time period distinct from the first time period. A duration of the first time period and/or the second time period may be at least 10 minutes, at least 20 minutes, at least 30 minutes, or at least 60 minutes. A duration of the first time period and/or the second time period may be less than a week, less than 24 hours, less than 12 hours, less than 6 hours, less than 2 hours, or less than one hour. For example, the second timing sequence may enable operation of the first plurality of SLE's solely between midnight and noon and may enable operation of the third plurality of SLE's solely between noon and midnight, *e.g.* to effect irradiation of an udder with near infrared light of a wavelength of 800-1100 nm during a morning milking and irradiation of the udder with near ultraviolet light of a wavelength of 400-600 nm during an evening milking. Similarly, the second timing sequence may enable operation of the third plurality of SLE's solely one day per week.

The irradiation device may comprise a magnetic position sensor. The irradiation device may be structured to control operation of any of the light emitters as a function of location, *e.g.* as a function of an output of the magnetic position sensor. The magnetic position sensor and/or the controller may store location data that may be compared with an output of the magnetic position sensor, *e.g.* to determine whether the irradiation device is in a known location. The irradiation device may be structured to transition to an "ENABLED" state in response to a signal from the magnetic position sensor indicative that the irradiation device is at a first location. The irradiation device may be structured to transition to a "DISABLED" state in response to a signal from the magnetic position sensor indicative that the irradiation device is at a second location. In the "DISABLED" state, the irradiation device may interrupt a supply of power to any of the light emitters. In the "ENABLED" state, the irradiation device may permit a supply of power to any of the light emitters.

The irradiation device may comprise an interface, *e.g.* a wired and/or wireless interface, to receive instructions and/or data to alter any of the stored location data, the first timing sequence, the second timing sequence, the first, second, third, and fourth lengths of time, the first period of time, and the second period of time. The interface may comprise a receptacle, *e.g.* a receptacle conforming with the USB-C standard, and/or a radio transceiver, *e.g.* a radio transceiver conforming with the Bluetooth standard.

The irradiation device may be structured to protect the power supply system and/or the first plurality of SLE's and/or the fourth plurality of SLE's and/or the additional SLE's and/or the other additional SLE's and/or the detector against an ingress of liquid at a rating of at least IPX5 according to international standard IEC 60529. The irradiation device may be structured to protect the power supply system and/or the first plurality of SLE's and/or the fourth plurality of SLE's and/or the additional SLE's and/or the other additional SLE's and/or the detector against an ingress of liquid at a rating of at least IPX6, at least IPX6K, at least IPX9, or at least IPX9K according to international standard IEC 60529. The irradiation device may comprise a housing, *e.g.* a polymeric housing. The housing may be mounted to and at least partially enclose the support. The housing may constitute an outermost surface of the irradiation device. The housing may serve to inhibit an ingress of liquid as discussed above.

The irradiation device may comprise a first window, *e.g.* a first sheet of laminated glass or a first sheet of transparent plastic material, adjacent the first surface. The first window may form a watertight seal with the housing. The first window may transparently close a first opening in the housing, *e.g.* against an ingress of liquid as discussed above. The first window may constitute an outermost surface of the irradiation device. The irradiation device may comprise a second window, *e.g.* a second sheet of laminated glass or a second sheet of transparent plastic material, adjacent the second surface. The second window may form a watertight seal with the housing. The second window may transparently close a second opening in the housing, *e.g.* against an ingress of liquid as discussed above. The second window may constitute an outermost surface of the irradiation device. The irradiation device may be structured to withstand a compressive force arising from a dairy animal such as a cow or camel compressing the irradiation device against a rigid wall, *e.g.* by leaning on, actively pushing against, or kicking (a major surface of) the irradiation device. The irradiation device may comprise a metallic heat sink. The heat sink may comprise a plurality of fins that constitute an outermost surface of the irradiation device. The heat sink may constitute a portion of the support, *e.g.* a portion of the main body. The support may be shaped to engage and/or receive a portion of an ambient structure, *e.g.* for the sake of positioning and/or fastening the irradiation device to the ambient structure. The support may be shaped to clampingly engage a portion of an ambient fencing. For example, the support may comprise a two-part main body, the individual parts of which jointly form at least one cylindrical passage suited to clampingly receive a portion of a tubular fencing when the individual parts are bolted together. The irradiation device may comprise at least one fastener. The at least one fastener may constitute all or part of an assembly that attaches the irradiation device to an ambient structure such as a fence, gate, or wall. The at least one fastener may be selected from the group consisting of a screw, a bolt, an internally threaded component, a ring, a strap, and a component comprising a mounting hole. A portion of the support may constitute the internally threaded component and/or the component comprising a mounting hole.

The irradiation device may comprise a first positioning system. An orientation of the first surface may be adjustable in one or two dimensions relative to another portion of the irradiation device, *e.g.* relative to a main body or housing of the irradiation device, by means of the first positioning system. The first positioning system may support the first surface, *e.g.* a printed circuit board or other portion of the support comprising the first surface, such that an orientation of the first surface is adjustable in one or two dimensions relative to another portion of the irradiation device, *e.g.* relative to a main body or housing of the irradiation device. The first positioning system may adjust an orientation of the first surface to irradiate an udder. The first positioning system may comprise at least one motor, the at least on motor providing a motive force necessary to adjust an orientation of the first surface relative to another portion of the irradiation device. The controller may use signals obtained from a detector, *e.g.* an udder detector and/or a teat detector, to control the at least one motor such that at least 80% of a light emitted by the first plurality of SLE's and/or the additional SLE's irradiate an udder. The controller may use signals obtained from the detector to control the at least one motor such that the first direction points toward a major surface of an udder, *e.g.* toward a center of an at least 10 cm x 10 cm surface of an udder. The detector, *e.g.* the udder detector and/or the teat detector, may be an element of a milking system as described below.

The irradiation device may comprise a second positioning system. An orientation of the second surface may be adjustable in one or two dimensions relative to another portion of the irradiation device, *e.g.* relative to a main body or housing of the irradiation device, by means of the second positioning system. The second positioning system may support the second surface, *e.g.* a printed circuit board or other portion of the support comprising the second surface, such that an orientation of the second surface is adjustable in one or two dimensions relative to another portion of the irradiation device, *e.g.* relative to a main body or housing of the irradiation device. The second positioning system may adjust an orientation of the second surface to irradiate an udder. The second positioning system may comprise at least one motor, the at least on motor providing a motive force necessary to adjust an orientation of the second surface relative to another portion of the irradiation device. The controller may use signals obtained from a detector, *e.g.* an udder detector and/or a teat detector, to control the at least one motor such that at least 80% of a light emitted by the fourth plurality of SLE's and/or the other additional SLE's irradiate an udder. The controller may use signals obtained from the detector to control the at least one motor such that the second direction points toward a major surface of an udder, *e.g.* toward a center of an at least 10 cm x 10 cm surface of an udder. The detector, *e.g.* the udder detector and/or the teat detector, may be an element of a milking system as described below.

An imaginary, minimally-sized rectangular parallelepiped cuboid enclosing the main body of the irradiation device or enclosing the irradiation device (excepting the power cord and/or the at least one fastener) may be less than 80 cm in length and/or less than 50 cm in height and/or less than 30 cm in thickness. An imaginary, minimally-sized rectangular parallelepiped cuboid enclosing the main body of the irradiation device or enclosing the irradiation device (excepting the power cord and/or the at least one fastener) may be less than 40 cm in length and/or less than 30 cm in height and/or less than 15 cm in thickness. In the standard operating state of the irradiation device, the length and thickness of the irradiation device may be oriented horizontally and the height of the irradiation device may be oriented vertically. The at least one fastener may be structured and arranged such that the length and thickness of the irradiation device are oriented horizontally and the height of the irradiation device is oriented vertically in the standard operating state of the irradiation device.

The present disclosure discloses an irradiation system. The irradiation system may comprise at least one irradiation device, *e.g.* at least one (laser) irradiation device as disclosed above. The irradiation system may comprise a positioning system. A portion of the positioning system, *e.g.* an attachment portion, may be rigidly fastened to an ambient structure, *e.g.* to fencing or a wall. The positioning system may support the irradiation device such that the irradiation device is movable in one, two, or three dimensions relative to the ambient structure. The positioning system may position the irradiation device to irradiate an udder. The positioning system may comprise at least one motor, the at least on motor providing a motive force necessary to move the irradiation device in one, two, or three dimensions relative to the ambient structure. The positioning system may comprise at least one udder detector, *e.g.* a detector structured to detect a location of an udder. The at least one udder detector may comprise at least one of an udder detector of the irradiation device, a camera, a scanning laser, a sonar emitter, and a sonar detector. The positioning system may comprise a controller, *e.g.* a controller of the irradiation device. The controller may use signals obtained from a detector, *e.g.* from a teat detector and/or the at least one udder detector to control the at least one motor such that at least 80% of a light emitted by the irradiation device irradiates an udder. The controller may use signals obtained from a detector, *e.g.* from a teat detector and/or the at least one udder detector to control the at least one motor such that the first direction points toward a major surface of an udder, *e.g.* toward a center of an at least 10 cm x 10 cm surface of an udder. The detector, *e.g.* the udder detector and/or the teat detector, may be an element of a milking system as described below. The positioning system may be structured to withstand a force arising from a dairy animal leaning on, actively pushing against, or kicking the positioning system and/or the irradiation device. The positioning system may comprise at least one track. The positioning system may have an overall size of less than 100 cm x 60 cm x 50 cm or an overall size of less than 100 cm x 60 cm x 20 cm.

The present disclosure discloses a milking system. The milking system may comprise a first milking cup, *e.g.* a first milking cup to receive a first teat of an udder. The milking system may comprise a second milking cup, *e.g.* a second milking cup to receive a second teat of the udder. The milking system may comprise a third milking cup to receive a third teat of the udder. The milking system may comprise a fourth milking cup to receive a fourth teat of the udder. The udder may be an udder of a dairy animal such as a cow, goat, sheep, lama, or camel.

The milking system may comprise a robotic positioner, *e.g.* a(n articulated) robotic arm. The robotic positioner may be structured to engage the first milking cup to the first teat. The robotic positioner may be structured to engage the second, third, and fourth milking cups to the second, third, and fourth teats, respectively. The milking system may comprise a sensor, *e.g.* an optical teat detector, structured to detect a position of at least one of the first teat and the second teat. The sensor may be situated on the robotic arm. The sensor may emit a scanning beam of light. The sensor may detect a reflected portion of the emitted scanning beam of light to detect a position of at least one of the first teat and the second teat. The sensor may comprise a camera. The sensor may comprise a processor and an image processing software that, when executed on the processor, processes at least one image obtained from a camera to detect a position of at least one of the first teat and the second teat. The robotic positioner may use a signal from the sensor indicative of a position of the first teat to engage the first milking cup to the first teat. The robotic positioner may use a signal from the sensor indicative of a position of the second teat to engage the second milking cup to the second teat. The robotic positioner may comprise a tie string that ties the first milking cup to the robotic positioner, *e.g.* to prevent the first milking cup from falling to the ground and/or to permit retraction of the first milking cup to the robotic positioner. The robotic positioner may comprise at least one additional tie string for tying any of the second, third, and fourth milking cup to the robotic positioner.

The milking system may comprise a carousel, *e.g.* a carousel structured to transport a plurality of dairy animals. The carousel may be a milking carousel structured to transport a plurality of dairy animals during a milking operation. The carousel may be structured to transport the first milking cup and the second milking cup together with an individual dairy animal (of the plurality of dairy animals). The milking system may comprise an outer perimeter fence, *e.g.* in the form of fencing, tubing, or a wall. With the possible exception of gaps narrower than 50 cm, narrower than 30 cm, narrower than 20 cm, or narrower than 10, the outer perimeter fence may extend from a perimeter of an entrance corral where dairy animals enter the carousel to a perimeter of an exit corral where dairy animals exit the carousel. The outer perimeter fence may occlude at least 210°, at least 240°, at least 270°, at least 300°, or at least 330° of a circumference of the carousel.

A circle defined by a perimeter of the carousel may be subdivided into a set of non-overlapping circular sectors. The set may comprise a first circular sector corresponding to a section of the carousel adjacent the entrance corral. The set may comprise a second circular sector corresponding to a section of the carousel where dairy animals are prepared for milking, *e.g.* by attaching milking cups to the teats of the dairy animals. The set may comprise a third circular sector corresponding to a section of the carousel where the milking cups are removed from the dairy animals. The set may comprise a fourth circular sector corresponding to a section of the carousel adjacent the exit corral. The set may comprise a fifth circular sector corresponding to a section of the carousel where the dairy animals undergo milking. The aforementioned five circular sectors may constitute the entire set. Similarly, the set may comprise at least one further circular sector. The aforementioned five circular sectors may be stationary relative to an ambient environment of the milking carousel. The second circular sector may have a central angle of not less than 45°, not less than 30°, not less than 20°, or not less than 15°. The second circular sector may have a central angle of not more than 60°, not more than 45°, not more than 30°, or not more than 20°. The fourth circular sector may have a central angle of not less than 45°, not less than 30°, not less than 20°, not less than 15°, or not less than 10°. The fourth circular sector may have a central angle of not more than 60°, not more than 45°, not more than 30°, or not more than 20°.

The circle defined by a perimeter of the carousel may be subdivided into two non-overlapping circular sectors, namely a sixth circular sector and a seventh circular sector. The sixth circular sector and the seventh circular sector may be stationary relative to an ambient environment of the milking carousel.

The milking system may comprise at least one milking stall. The milking stall may be structured to confine an individual dairy animal. The milking stall may restrict the position of an individual dairy animal such that the udder of the individual dairy animal is situated within 25 cm of a target position in a longitudinal direction and within 15 cm of a target position in a lateral direction. The milking stall may restrict the position of an individual dairy animal such that the udder of the individual dairy animal is situated within 15 cm of a target position in a longitudinal direction and within 5 cm of a target position in a lateral direction. The milking stall may comprise fencing, tubing, or a wall that restricts the position of an individual dairy animal in a first lateral direction. The milking stall may comprise fencing, tubing, a wall, or a gate that restricts the position of the individual dairy animal in a second lateral direction. The milking stall may comprise fencing, tubing, a trough, a wall, or a gate that restricts the position of the individual dairy animal in a forward direction. The milking stall may comprise fencing, tubing, a pusher, a wall, or a gate that restricts the position of the individual dairy animal in a rearward direction. The milking system may comprise fencing, tubing, or a wall that restricts the position of the individual dairy animal in a milking stall in a rearward direction. For example, the outer perimeter fence (ambient to the milking carousel) may restrict the position of the individual dairy animal in a milking stall (on the milking carousel) in a rearward direction. The milking system may comprise an RFID reader, *e.g.* for reading an RFID tag worn by a dairy animal. The RFID reader may output a signal indicative of the presence of a dairy animal in the milking stall. The RFID reader may output a signal indicative of an absence of a dairy animal in the milking stall.

The milking system may comprise at least one irradiation device, *e.g.* at least one (laser) irradiation device as disclosed above. For example, the milking stall may comprise an irradiation device mounted on a first lateral side of the milking stall, *e.g.* on fencing, tubing, a wall, or a gate that constitutes a first lateral side of the milking stall. Similarly, the milking stall may comprise an irradiation device mounted on a second lateral side of the milking stall, *e.g.* on fencing, tubing, a wall, or a gate that constitutes a second lateral side of the milking stall. Similarly, the milking stall may comprise a irradiation device mounted on a rear side of the milking stall, *e.g.* on fencing, tubing, a wall, or a gate that constitutes a rear side of the milking stall. The irradiation device may be mounted on the milking stall such that a volume irradiated by the first SLE's coincides with a typical udder position of a dairy animal confined in the milking stall. The irradiation device may be mounted on the milking stall such that a volume irradiated by the fourth SLE's coincides with a typical udder position of a dairy animal confined in the milking stall. The irradiation device may be mounted on the milking stall such that an upper edge of the irradiation device is no less than 30 cm and no more than 60 cm above the floor of the milking stall.

The milking system may comprise a plurality of irradiation devices mounted on the outer perimeter fence (ambient to the milking carousel). The respective irradiation device may be mounted on the outer perimeter fence such that a volume irradiated by the first SLE's coincides with a typical udder position of a dairy animal confined in an adjacent milking stall. The respective irradiation device may be mounted on the outer perimeter fence such that an upper edge of the irradiation device is no less than 30 cm and no more than 60 cm above the floor of an adjacent milking stall. The plurality of irradiation devices may define an eighth circular sector. The eighth circular sector may be a smallest circular sector of a circle defined by the outer perimeter fence, which circular sector encloses the plurality of irradiation devices. The respective first surfaces of the plurality of irradiation devices may collectively occupy at least 30%, at least 50%, or at least 80% of an arc length of the sixth circular sector. The eighth circular sector may be devoid of overlap with the first circular sector and the fourth circular sector. The eighth circular sector may be devoid of overlap with the second circular sector and/or the third circular sector. The eighth circular sector may overlap an entirety of the fifth circular sector. Similarly, the fifth circular sector may overlap an entirety of the eighth circular sector falling within the circle defined by a perimeter of the carousel.

The plurality of irradiation devices may be positioned such that the sixth circular sector meets any of the following criteria. The sixth circular sector may have a central angle of not less than 90°, not less than 120°, not less than 150°, not less than 180°, or not less than 210°. The sixth circular sector may have a central angle of not more than 120°, not more than 150°, not more than 180°, not more than 210°, not more than 240°, or not more than 270°. As measured in a direction of rotation of the milking carousel from a radius aligned with a beginning of an entrance for dairy animals into the milking carousel, the bounds of the sixth circular sector may be as follows. The sixth circular section may begin at an angle of at least 45°, at least 60°, at least 90°, or at least 120°. The sixth circular section may begin at an angle of not more than 150°, not more than 120°, not more than 90°, or not more than 60°. The sixth circular section may conclude at an angle of at least 210°, at least 240°, at least 270°, or at least 300°. The sixth circular section may conclude at an angle of not more than 240°, not more than 270°, not more than 300°, or not more than 315°.

The milking system may comprise a detector. The detector may be structured to distinguish between a presence and an absence of an irradiation target. For example, the detector may be structured to distinguish between a presence and an absence of a dairy animal in a milking stall. The detector may comprise an infrared sensor. The detector may comprise a time-of-flight detector. The detector may comprise an RFID reader, *e.g.* as described above. The detector may comprise a camera. The detector may comprise a processor and an image processing software that, when executed on the processor, processes at least one image obtained from a camera to provide at least one of an output indicative of a presence of a dairy animal (in the at least one camera image) and an output indicative of an absence of a dairy animal (in the at least one camera image). The detector may comprise a switch. The switch may be situated on a floor, a front side, a lateral side, or a rear side of the milking stall. The switch may be structured to adopt a first operating state in the presence of a dairy animal in the milking stall. The switch may be structured to adopt a second operating state in the absence of a dairy animal in the milking stall. The switch may be a mechanic switch.

The milking system may comprise a controller, *e.g.* a milking controller. The controller may be structured to connect a supply of power to an irradiation device in response to a signal from a detector indicative of a presence of an irradiation target, *e.g.* a presence of a dairy animal in a milking stall in an irradiation zone of the irradiation device. The controller may be structured to interrupt a supply of power to an irradiation device in response to a signal from a detector indicative of an absence of an irradiation target, *e.g.* an absence of a dairy animal in a milking stall in an irradiation zone of the irradiation device. Similarly, the controller may effect connection / interruption of a supply of power to the first plurality of SLE's and/or the additional SLE's in response to a signal from a detector indicative of a presence / an absence of an irradiation target, *e.g.* a presence / an absence of a dairy animal in a milking stall in an irradiation zone of the first plurality of SLE's. The controller may effect connection / interruption of a supply of power to the fourth plurality of SLE's and/or the other additional SLE's in response to a signal from a detector indicative of a presence / an absence of an irradiation target, *e.g.* a presence / an absence of a dairy animal in a milking stall in an irradiation zone of the fourth plurality of SLE's. The detector may be a component of the controller. For example, the controller may comprise a detector, *e.g.* as described above.

The milking system may be structured to connect / interrupt a supply of power to a irradiation device as a function of an angular position of the irradiation device and/or as a function of an angular position of the milking carousel. The milking system may connect a supply of power to any individual irradiation device situated and/or deemed to be situated in the sixth circular sector. The milking system may interrupt a supply of power to any individual irradiation device situated and/or deemed to be situated in a seventh circular sector. The milking system may comprise an angular position sensor that outputs a signal indicative of an angular position of the carousel. The milking system may use the signal output by the angular position sensor to assess an angular position of any individual irradiation device, *e.g.* to assess whether the respective irradiation device is situated in the sixth / seventh circular sector, and to connect / interrupt a supply of power to the respective irradiation device, accordingly. The milking system may comprise a plurality of switches. The milking system may be structured such that, for each of the plurality of switches individually, the respective switch connects / interrupts a supply of power to at least one irradiation device, *e.g.* to at least one irradiation device in a proximity (within one meter, for example) of the respective individual switch. The milking system may be structured such that, for each of the plurality of switches individually, the respective switch is in a first state when the respective switch is in the sixth circular sector. The milking system may be structured such that, for each of the plurality of switches individually, the respective switch is in a second state when the respective switch is in the seventh circular sector. The first state may connect a supply of power to the respective at least one irradiation device. The second state may interrupt a supply of power to the respective at least one irradiation device. The teachings of this paragraph may apply exclusively to irradiation devices mounted to and rotating with the carousel.

The milking system may comprise a milk tester. The milk tester may be in fluid connection with any of the first, second, third, and fourth milking cups. The milk tester may measure at least one parameter of milk milked from the udder of a dairy animal, *e.g.* via the respective milking cup(s). The milking system may adjust a spectrum of light emitted by the irradiation device, *i.e.* by the light emitters, as a function of the measured parameter(s). The third plurality of SLE's may comprise a seventh plurality of SLE's that emit light at a wavelength greater than 400 nm and less than 450 nm. The milking system may be structured to connect a supply of power to the seventh plurality of SLE's in response to a signal indicative that a dairy animal in an irradiation zone of the seventh plurality of SLE's has a bacterial infection. The milking system may comprise a sensor that assesses a somatic cell count in milking obtained from the dairy animal. The sensor may output a signal indicative that the dairy animal has a bacterial infection if the assessed somatic cell count exceeds a stored threshold, *e.g.* a threshold of 100,000 per milliliter. The milking system may store data indicative of a health of individual dairy animals. The controller may output a signal indicative that the dairy animal has a bacterial infection if data stored for a dairy animal identified by an RFID reader is indicative of a bacterial infection.

As measured in a direction of rotation of the milking carousel from a radius aligned with a beginning of an entrance for dairy animals into the milking carousel, the bounds of the sixth circular sector may be as follows. The sixth circular section may begin at an angle of at least 45°, at least 60°, at least 90°, or at least 120°. The sixth circular section may begin at an angle of not more than 150°, not more than 120°, not more than 90°, or not more than 60°. The sixth circular section may conclude at an angle of at least 210°, at least 240°, at least 270°, or at least 300°. The sixth circular section may conclude at an angle of not more than 240°, not more than 270°, not more than 300°, or not more than 315°. The sixth circular sector may overlap an entirety of the eighth circular sector falling within the circle defined by a perimeter of the carousel. Similarly, the eighth circular sector may overlap an entirety of the sixth circular sector. The union of the first circular sector, the second circular sector, the third circular sector, and the fourth circular sector may constitute the sixth circular sector.

The milking system may comprise an interface, *e.g.* a wired and/or wireless interface, to receive instructions and/or data to alter data within the milking system defining any the first through seventh circular sections. The interface may comprise a receptacle, *e.g.* a receptacle conforming with the USB-C standard, and/or a radio transceiver, *e.g.* a radio transceiver conforming with the Bluetooth standard.

The milking system may comprise a pump and a tank. The milking system may comprise a first conduit that connects the first milking cup to the tank. The milking system may comprise a second conduit that connects the second milking cup to the tank. The pump may create suction in the first conduit, which suction extracts milk from the first teat via the first milking cup. The pump may create suction in the second conduit, which suction extracts milk from the second teat via the second milking cup. The first conduit may comprise a hose and/or piping. Similarly, the second conduit may comprise a hose and/or piping.

The milking system may comprise a power supply system. The power supply system may comprise a voltage converter, *e.g.* a voltage converter that converts a line voltage of 110 V or 220 V to a voltage of less than 40 V, or less than 20 V. The power supply system may comprise a network of power cords that connect a plurality of irradiation devices to the voltage converter. The power supply system may be structured such that electrical power supplied to the irradiation devices via the network of power cords has a voltage of less than 40 V, less than 20 V, or less than 15 V. The power supply system may comprise at least one slip ring and at least one brush that electrically contacts the slip ring. The at least one slip ring and the at least one brush may constitute a portion of an electrical path between the carousel and an ambient environment, *e.g.* between the carousel and the voltage converter. The power supply system may be structured such that electrical power supplied to the carousel to power the irradiation devices mounted on the carousel has a voltage of less than 40 V, less than 20 V, or less than 15 V.

The present disclosure discloses a first method. The first method may comprise equipping a milking system with an irradiation device. The milking system may be a milking system as disclosed above. The irradiation device may be an irradiation device as disclosed above. The first method may comprise mounting an irradiation device on a milking stall of the milking system as disclosed above.

The present disclosure discloses a second method. The second method may comprise using an irradiation device to bathe a surface of an udder in light at a wavelength greater than 400 nm and less than 1100 nm. The irradiation device may be an irradiation device as disclosed above. The second method may comprise using the irradiation device to bathe a surface of an udder such that the light-bathed udder surface has an area of least 100 cm², at least 150 cm², at least 200 cm², at least 300 cm², at least 400 cm², or at least 600 cm². The second method may comprise milking the udder using a milking system. The milking system may be a milking system as disclosed above. The second method may comprise using the irradiation device to bathe a surface of an udder in light during the milking.

The various embodiments of the present disclosure having been described above in general terms, the embodiments shown in the Figures will now be elucidated.

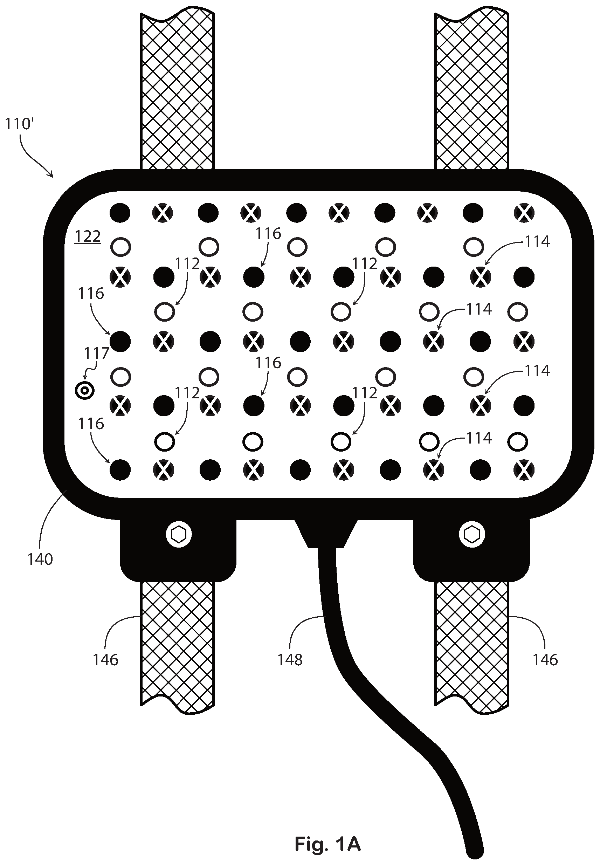

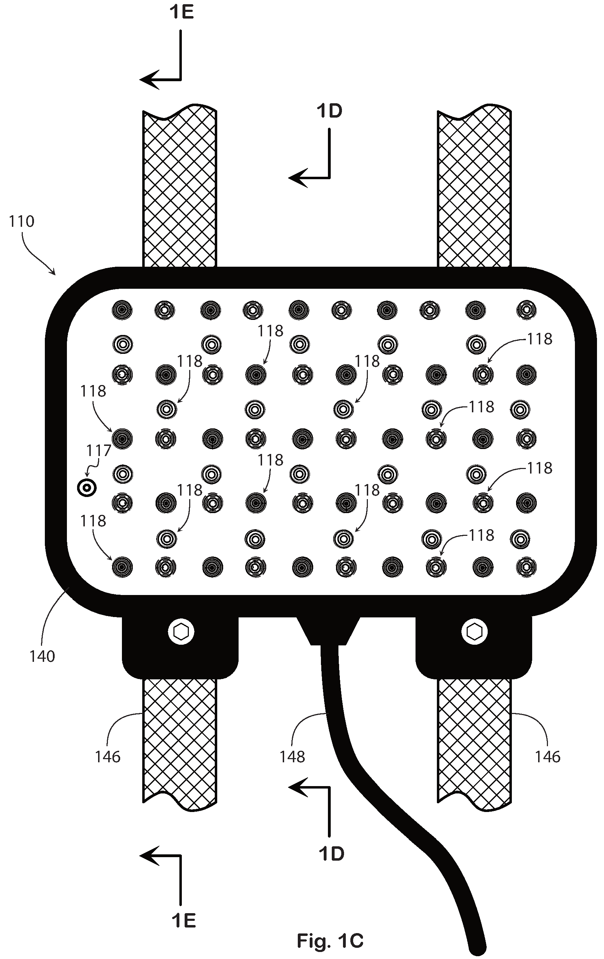

Figure 1A shows a schematic depiction of a laser irradiation device 110' in accordance with the present disclosure, *e.g.* as described above. In the depicted embodiment, laser irradiation device 110' is shown as comprising a plurality of laser diodes 112, a first plurality of LED's 114, a second plurality of LED's 116, a detector 117, a first surface 122, a housing 140, straps 146, and a power cord 148. The plurality of laser diodes 112, the first plurality of LED's 114, and the second plurality of LED's 116 are distributed on first surface 122. Detector 117 is also situated on first surface 122 and detects a presence of a dairy animal in an irradiation zone of the plurality of laser diodes 112. For the sake of better depiction, Figure 1A shows laser irradiation device 110' without the plurality of lenses present in Figures 1C-1E.

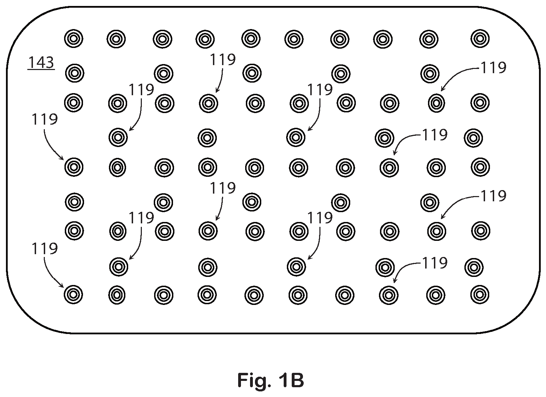

Figure 1B shows a schematic depiction of a plurality of lenses 119 distributed on a lens carrier 143 in accordance with the present disclosure, *e.g.* as described above.

Figure 1C shows a schematic depiction of laser irradiation device 110 of Fig. 1A including the plurality of lenses 119 of Figure 1B. Figure 1C depicts how the plurality of lenses 119 shown in Figure 1B individually align with laser diodes 112, LED's 114, and LED's 116 shown in Figure 1A to form a plurality of focussed SLE's 118.

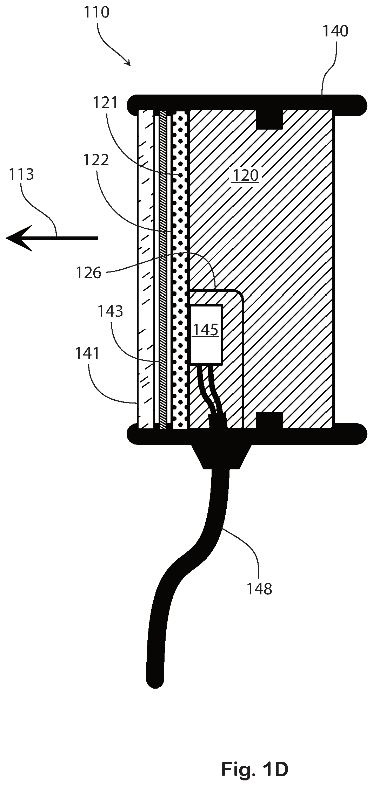

Figure 1D shows a schematic depiction of a first cross-section of laser irradiation device 110 of Fig. 1C. In the depicted embodiment, laser irradiation device 110 is shown as comprising a power supply 145, a main body 120, a printed circuit board 121, first surface 122 of printed circuit board 121, a first cutout 126, housing 140, a first window 141, lens carrier 143, and power cord 148. Figure 1B also depicts a first direction 113, *i.e.* a general direction of light emitted by laser diodes 112 and LED's 114, 116. Power supply 145 is depicted as being situated in first cutout 126 of main body 120.

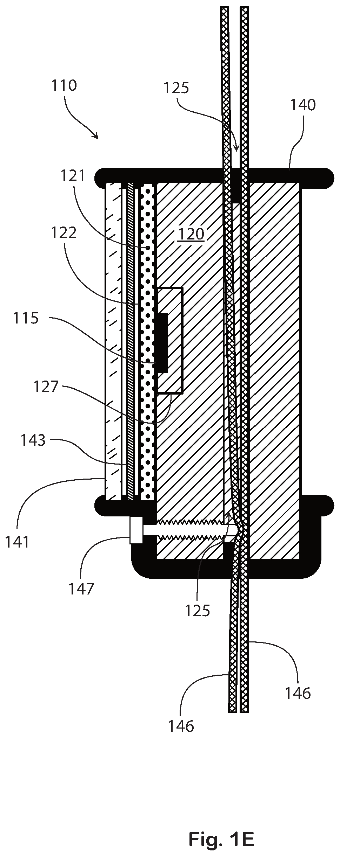

Figure 1E shows a schematic depiction of a second cross-section of laser irradiation device 110 of Fig. 1C. In the depicted embodiment, laser irradiation device 110 is shown as comprising a controller 115, main body 120, printed circuit board 121, first surface 122 of printed circuit board 121, a through hole 125, a second cutout 127, housing 140, first window 141, lens carrier 143, straps 146, and a screw 147. Controller 115 is depicted as being situated in second cutout 127 of main body 120. Straps 146 pass through openings in housing 140 and through hole 125 in main body 120 and are held in place by screw 147.

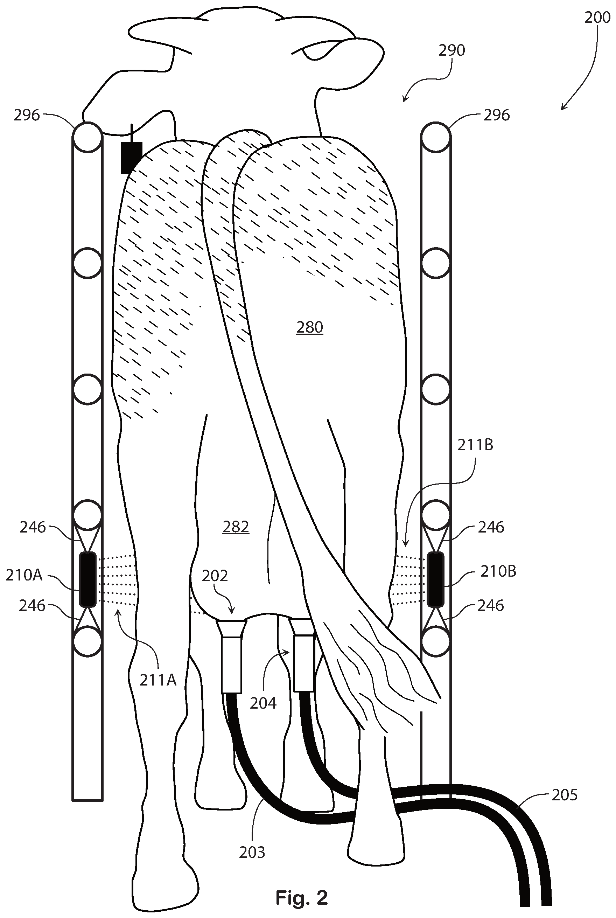

Figure 2 shows a schematic depiction of a milking system 200 in accordance with the present disclosure, *e.g.* as described above. In the depicted embodiment, milking system 200 is shown as comprising a first milking cup 202, a first hose 203, a second milking cup 204, a second hose 205, a first irradiation device 210A, and a second irradiation device 210B. In the depicted embodiment, first irradiation device 210A emits light 211A and second irradiation device 210B emits light 211B, which emitted light 211A, 211B irradiates a surface of an udder 282 of dairy animal 280 confined in a milking stall 290, *inter alia* by side fencing 296 that restricts a position of dairy animal 280 in a lateral direction. First irradiation device 210A and second irradiation device 210B are hung at an appropriate height to irradiate udder 282 by straps 246 between respective tubes of side fencing 296.

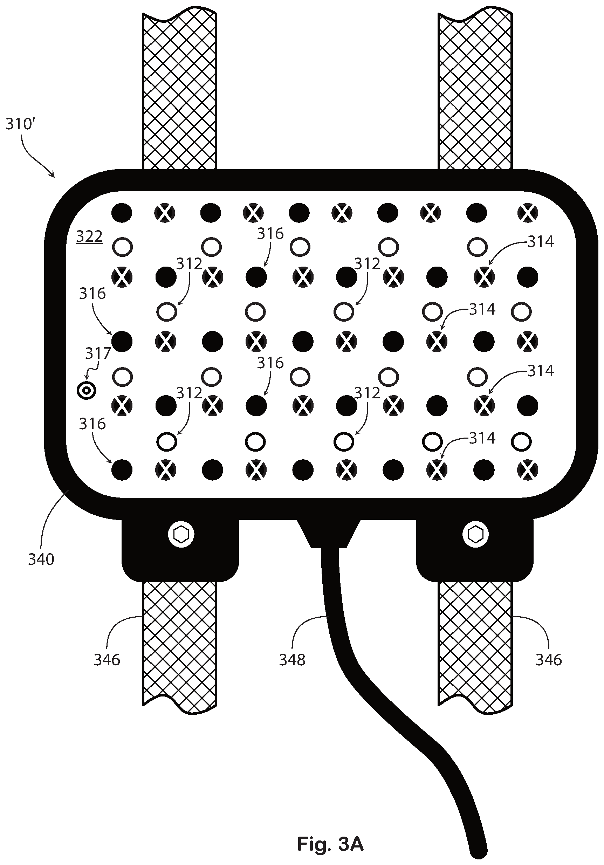

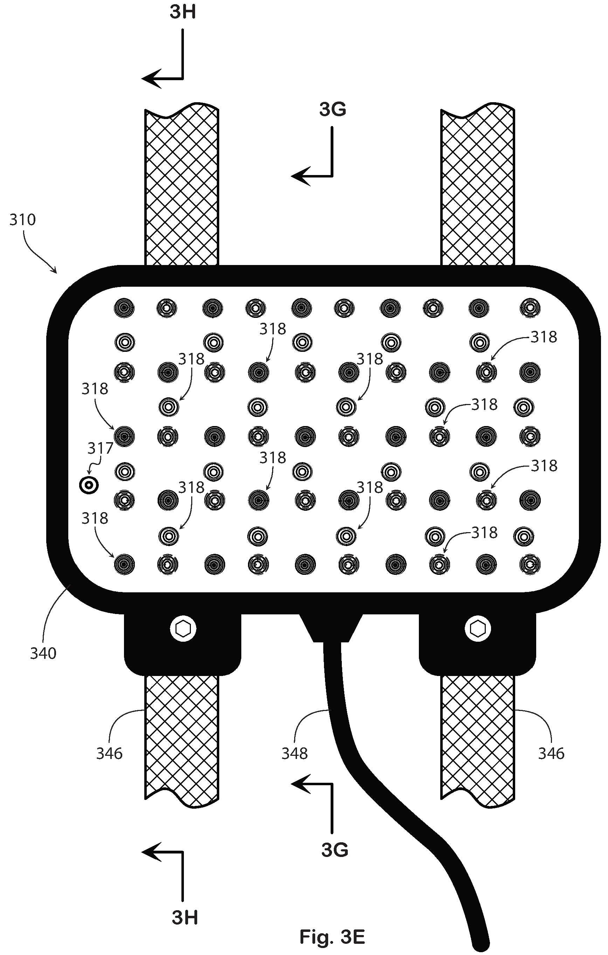

Figure 3A shows a schematic depiction of a first side of a laser irradiation device 310' in accordance with the present disclosure, *e.g.* as described above. In the depicted embodiment, laser irradiation device 310' is shown as comprising a first plurality of laser diodes 312, a first plurality of LED's 314, a second plurality of LED's 316, a first detector 317, a first surface 322, a housing 340, straps 346, and a power cord 348. The plurality of laser diodes 312, the first plurality of LED's 314, and the second plurality of LED's 316 are distributed on first surface 322. Detector 317 is also situated on first surface 322 and detects a presence of a dairy animal in an irradiation zone of laser diodes 312. For the sake of better depiction, Figure 3A shows laser irradiation device 310' without the first plurality of lenses present in Figures 3E-3H.

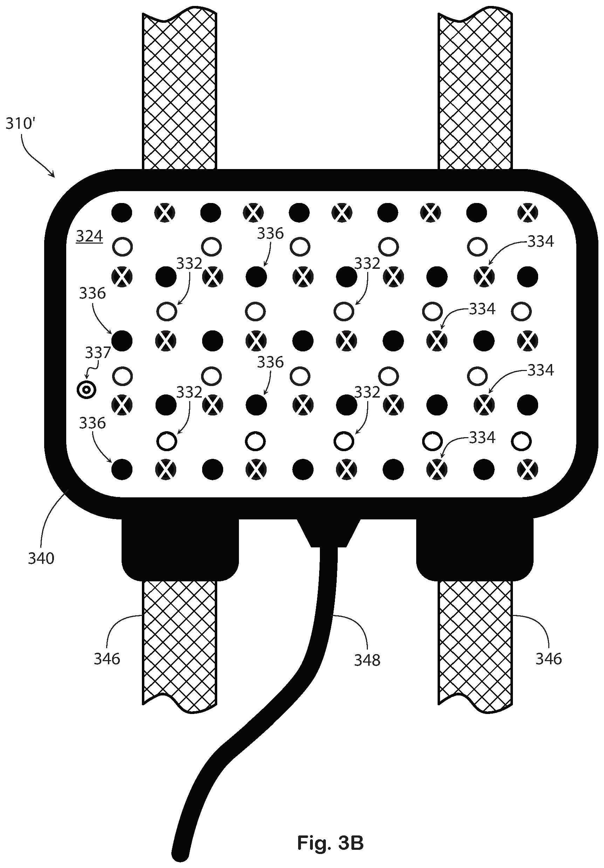

Figure 3B shows a schematic depiction of a second side of laser irradiation device 310' of Fig. 3A comprising housing 340, straps 346, and power cord 348. In the depicted embodiment, laser irradiation device 310' is shown as comprising a second surface 324, a second plurality of laser diodes 332, a third plurality of LED's 334, a fourth plurality of LED's 336, and a second detector 337. The second plurality of laser diodes 332, the third plurality of LED's 334, and the fourth plurality of LED's 336 are distributed on second surface 324. Detector 337 is also situated on second surface 324 and detects a presence of a dairy animal in an irradiation zone of laser diodes 332. For the sake of better depiction, Figure 3B shows laser irradiation device 310' without the second plurality of lenses present in Figures 3E-3H.

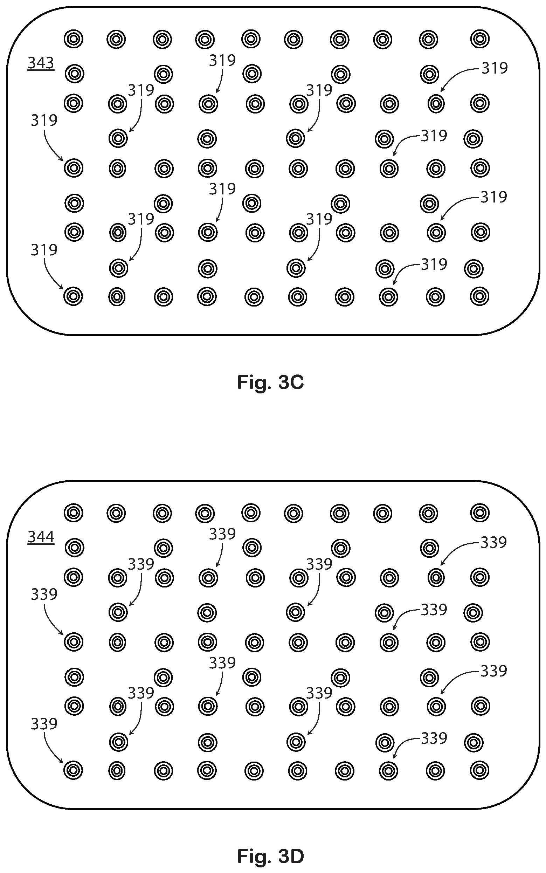

Figure 3C shows a schematic depiction of a first plurality of lenses 319 distributed on a first lens carrier 343 in accordance with the present disclosure, *e.g.* as described above.

Figure 3D shows a schematic depiction of a second plurality of lenses 339 distributed on a second lens carrier 344 in accordance with the present disclosure, *e.g.* as described above.

Figure 3E shows a schematic depiction of the first side of laser irradiation device 310 of Fig. 3A including the first plurality of lenses 319 of Figure 3C. Figure 3E depicts how the first plurality of lenses 319 shown in Figure 3C individually align with laser diodes 312, LED's 314, and LED's 316 shown in Figure 3A to form a first plurality of focussed SLE's 318.

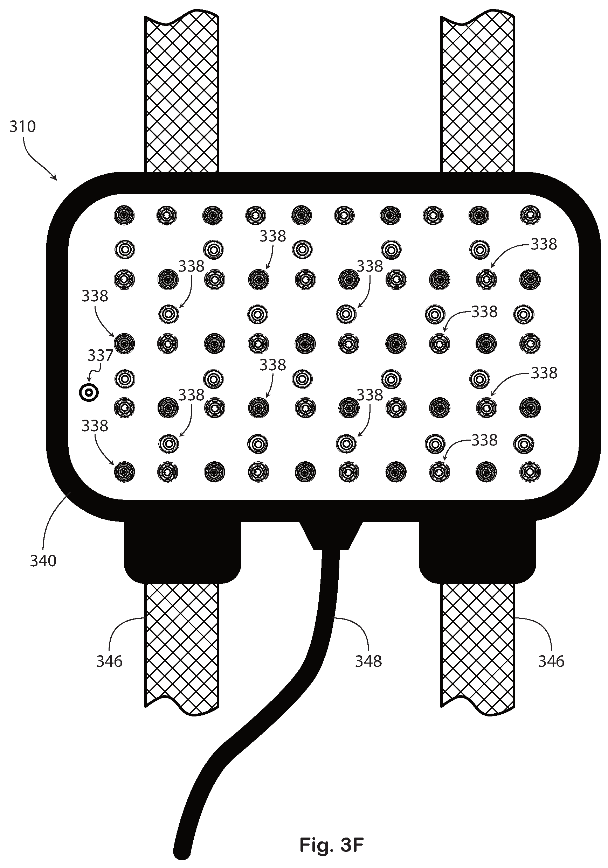

Figure 3F shows a schematic depiction of the second side of laser irradiation device 310 of Fig. 3B including the second plurality of lenses 339 of Figure 3D. Figure 3F depicts how the plurality of lenses 319 shown in Figure 3C individually align with laser diodes 332, LED's 334, and LED's 336 shown in Figure 3B to form a second plurality of focussed SLE's 338.

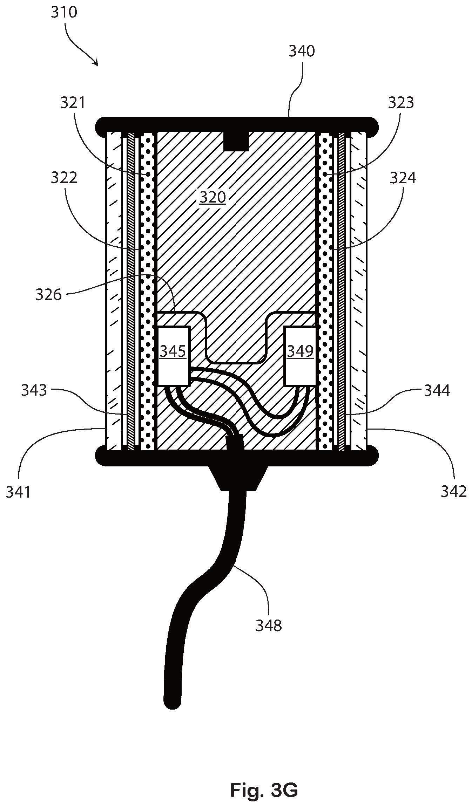

Figure 3G shows a schematic depiction of a first cross-section of laser irradiation device 310 of Fig. 3E. In the depicted embodiment, laser irradiation device 310 is shown as comprising a power supply 345, a main body 320, a first printed circuit board 321, first surface 322 of printed circuit board 321, a second printed circuit board 323, a second surface 324 of second printed circuit board 323, a first cutout 326, a connector 349, housing 340, a first window 341, a second window 342, first lens carrier 343, second lens carrier 344, and power cord 348. Power supply 345 and connector 349 are depicted as being situated in first cutout 326 of main body 320. Power supply 345 is mounted to first printed circuit board 321, and connector 349 is mounted to second printed circuit board 323.

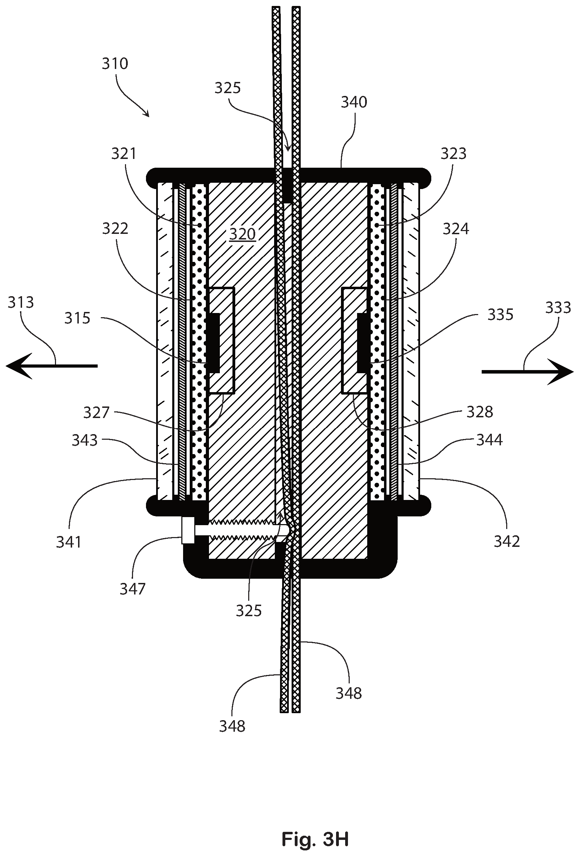

Figure 3H shows a schematic depiction of a second cross-section of laser irradiation device 310 of Fig. 3E comprising main body 320, first printed circuit board 321, first surface 322, second printed circuit board 323, second surface 324, housing 340, first window 341, second window 342, first lens carrier 343, second lens carrier 344, and straps 346. In the depicted embodiment, laser irradiation device 310 is shown as further comprising a first controller 315, a through hole 325, a second cutout 327, a third cutout 328, a second controller 335, and a screw 347. Controller 315 is depicted as being situated in second cutout 327 of main body 320. Straps 346 pass through openings in housing 340 and through hole 325 in main body 320 and are held in place by screw 347. Figure 3D also depicts a first direction 313, *i.e.* a general direction of light emitted by laser diodes 312 and LED's 314, 316, as well as a second direction 333, *i.e.* a general direction of light emitted by laser diodes 332 and LED's 334, 336. In the depicted embodiment, second surface 324 is parallel to first surface 322, and second direction 333 is antiparallel to first direction 313. First controller 315 controls a supply of power to laser diodes 312 and LED's 314, 316 in response to a signal from first detector 317, *e.g.* a signal indicative of whether a dairy animal is in an irradiation zone of laser diodes 312 and LED's 314, 316. Second controller 335 controls a supply of power to laser diodes 332 and LED's 334, 336 in response to a signal from first detector 337, *e.g.* a signal indicative of whether a dairy animal is in an irradiation zone of laser diodes 332 and LED's 334, 336.

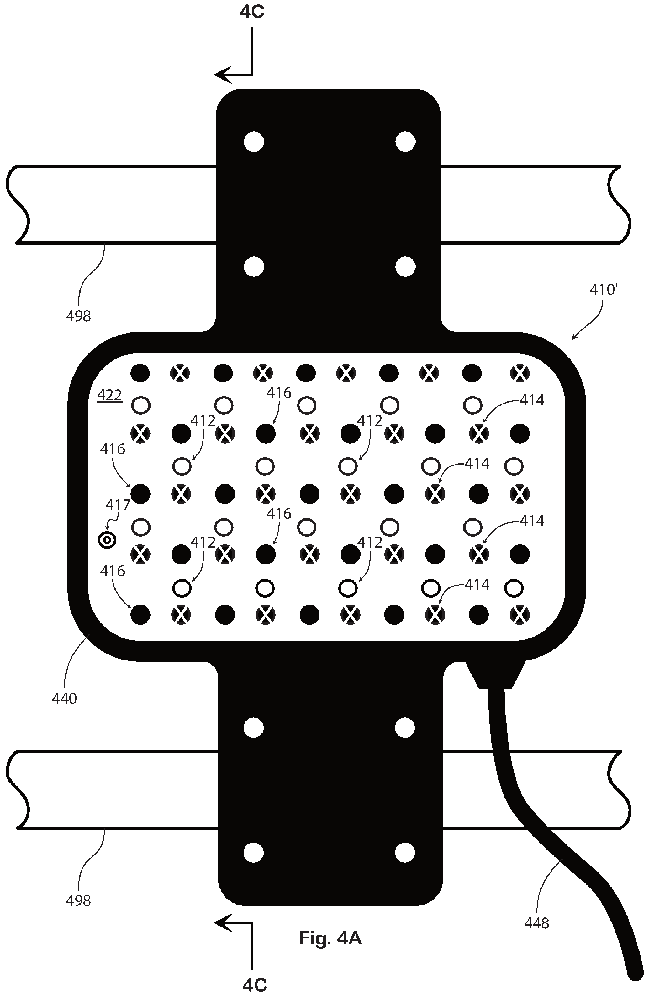

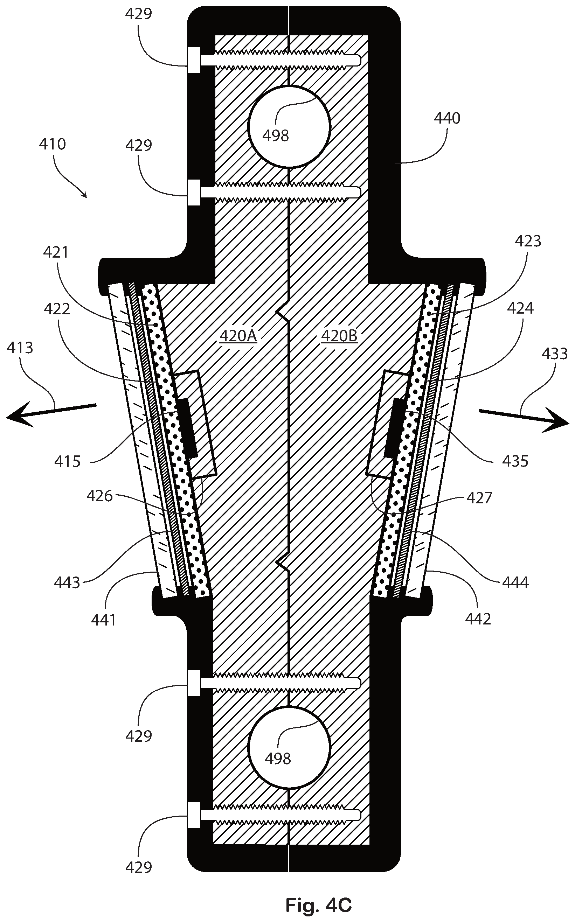

Figure 4A shows a schematic depiction of a first side of a laser irradiation device 410' in accordance with the present disclosure, *e.g.* as described above. In the depicted embodiment, laser irradiation device 410' is shown as comprising a first plurality of laser diodes 412, a first plurality of LED's 414, a second plurality of LED's 416, a first detector 417, a first surface 422, a housing 440, and a power cord 448. The plurality of laser diodes 412, the first plurality of LED's 414, and the second plurality of LED's 416 are distributed on first surface 422. Detector 417 is also situated on first surface 422 and detects a presence of a dairy animal in an irradiation zone of the first plurality of laser diodes 412. Laser irradiation device 410' is mounted to tubular fencing 498 of a milking stall. For the sake of better depiction, Figure 4A shows laser irradiation device 410' without lens carrier 443 present in Figure 4C.

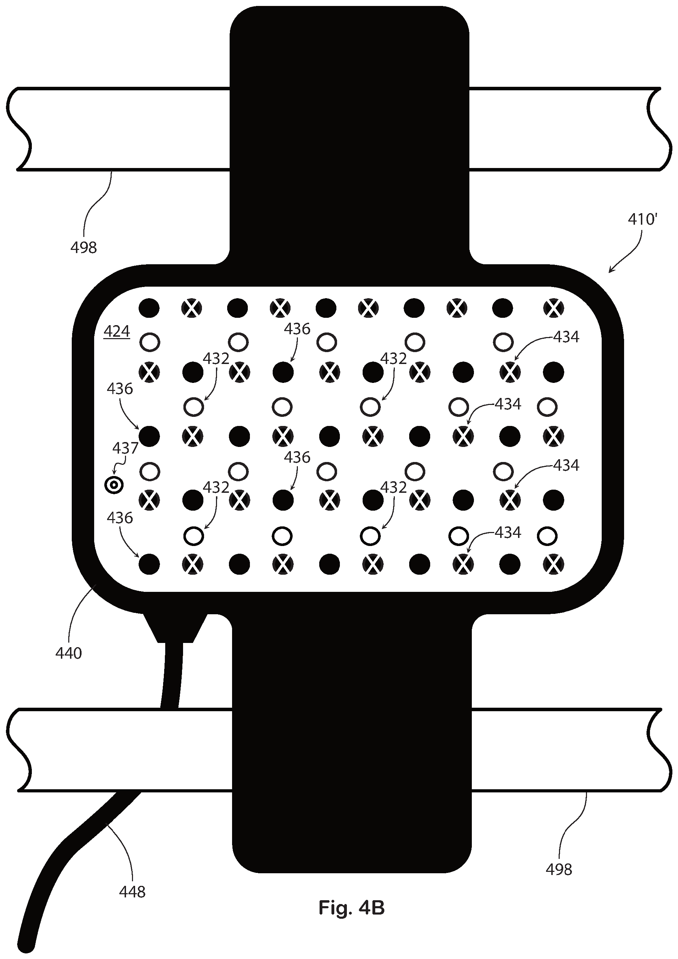

Figure 4B shows a schematic depiction of a second side of laser irradiation device 410' of Fig. 4A mounted to tubular fencing 498 and comprising housing 440 and power cord 448. In the depicted embodiment, laser irradiation device 410' is shown as further comprising a second surface 424, a second plurality of laser diodes 432, a third plurality of LED's 434, a fourth plurality of LED's 436, and a second detector 437. The second plurality of laser diodes 432, the third plurality of LED's 434, and the fourth plurality of LED's 436 are distributed on second surface 424. Detector 437 is also situated on second surface 424 and detects a presence of a dairy animal in an irradiation zone of the second plurality of laser diodes 432. For the sake of better depiction, Figure 4B shows laser irradiation device 410' without lens carrier 444 present in Figure 4C.

Figure 4C shows a schematic depiction of a cross-section of laser irradiation device 410 of Fig. 4A mounted to tubular fencing 498. In the depicted embodiment, laser irradiation device 410 is shown as comprising a first controller 415, a two-part main body, a first printed circuit board 421, first surface 422 of first printed circuit board 421, a second printed circuit board 423, second surface 424 of second printed circuit board 423, a through hole 425, a first cutout 426, a second cutout 427, screws 429, a second controller 435, housing 440, a first window 441, a second window 442, a first lens carrier 443, and a second lens carrier 444. First controller 415 is mounted to first printed circuit board 421 and situated in first cutout 426 of a first main body part 420A of the two-part main body. Second controller is mounted to second printed circuit board 423 and situated in second cutout 427 of a second main body part 420B of the two-part main body. First main body part 420A and second main body part 420B are bolted together by screws 429 and shaped to clampingly engage a portion of tubular fencing 498. Figure 4C also depicts a first direction 413, *i.e.* a general direction of light emitted by laser diodes 412 and LED's 414, 416, as well as a second direction 433, *i.e.* a general direction of light emitted by laser diodes 432 and LED's 434, 436. In the depicted embodiment, second surface 424 is angled by less than 20° relative to first surface 422, and second direction 433 is within 20° of opposite first direction 413 as measured in the depicted plane perpendicular to first surface 422. First controller 415 controls a supply of power to laser diodes 412 and LED's 414, 416 in response to a signal from first detector 417, *e.g.* a signal indicative of whether a dairy animal is in an irradiation zone of laser diodes 412 and LED's 414, 416. Second controller 435 controls a supply of power to laser diodes 432 and LED's 434, 436 in response to a signal from first detector 437, *e.g.* a signal indicative of whether a dairy animal is in an irradiation zone of laser diodes 432 and LED's 434, 436.

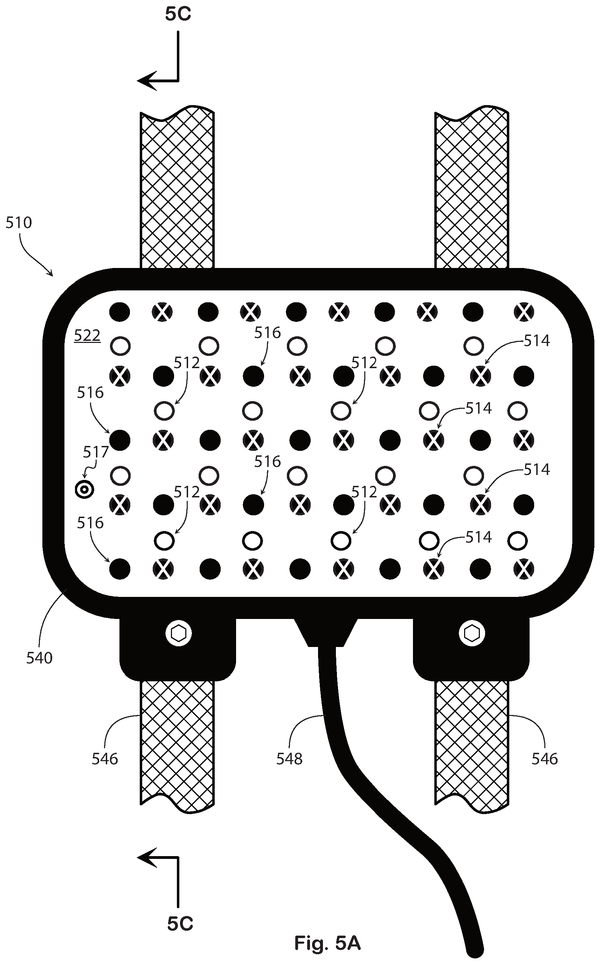

Figure 5A shows a schematic depiction of a first side of a laser irradiation device 510 in accordance with the present disclosure, *e.g.* as described above. In the depicted embodiment, laser irradiation device 510 is shown as comprising a first plurality of laser diodes 512, a first plurality of LED's 514, a second plurality of LED's 516, a first detector 517, a first surface 522, a housing 540, straps 546, and a power cord 548. The plurality of laser diodes 512, the first plurality of LED's 514, and the second plurality of LED's 516 are distributed on first surface 522. Detector 517 is also situated on first surface 522 and detects a presence of a dairy animal in an irradiation zone of laser diodes 512.

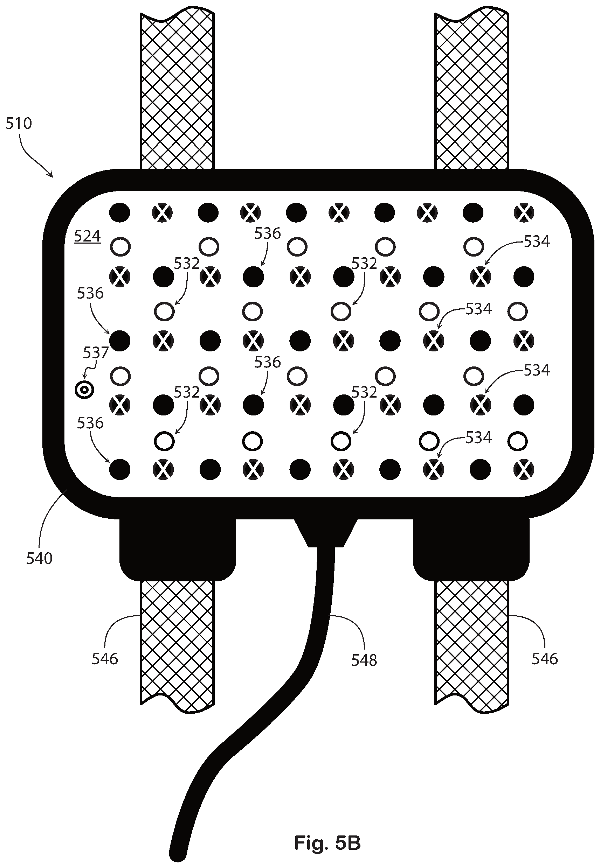

Figure 5B shows a schematic depiction of a second side of laser irradiation device 510 of Fig. 5A comprising housing 540, straps 546, and power cord 548. In the depicted embodiment, laser irradiation device 510 is shown as further comprising a second surface 524, a second plurality of laser diodes 532, a third plurality of LED's 534, a fourth plurality of LED's 536, and a second detector 537. The second plurality of laser diodes 532, the third plurality of LED's 534, and the fourth plurality of LED's 536 are distributed on second surface 524. Detector 537 is also situated on second surface 524 and detects a presence of a dairy animal in an irradiation zone of laser diodes 532.

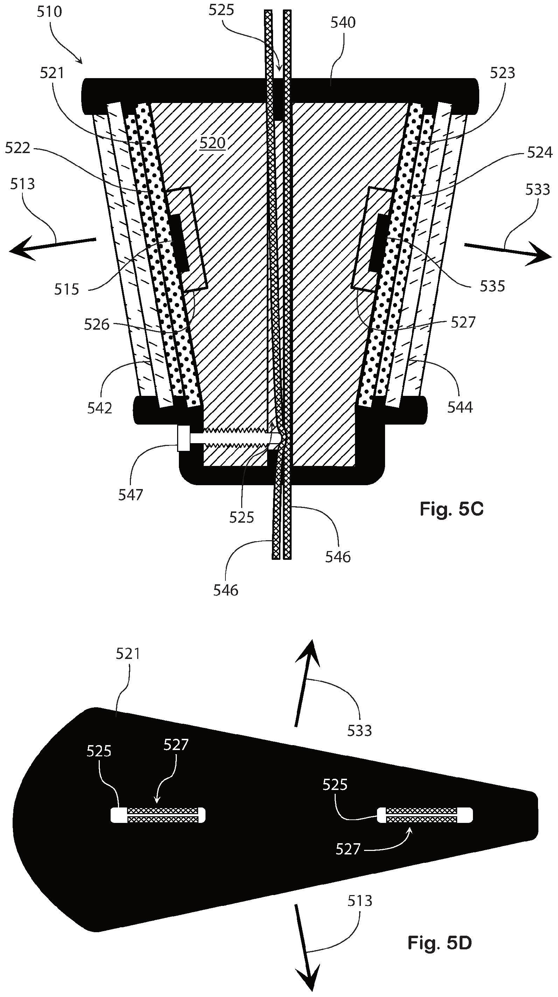

Figure 5C shows a schematic depiction of a second cross-section of laser irradiation device 510 of Fig. 5A. In the depicted embodiment, laser irradiation device 510 is shown as comprising a first controller 515, a main body 520, a first printed circuit board 521, first surface 522 of first printed circuit board 521, a second printed circuit board 523, second surface 524 of second printed circuit board 523, a through hole 525, a first cutout 526, a second cutout 527, a second controller 535, housing 540, a first window 541, a second window 542, straps 546, and a screw 547. First controller 515 is mounted to first printed circuit board 521 and situated in first cutout 526 of main body 520. Second controller 535 is mounted to second printed circuit board 523 and situated in first cutout 527 of main body 520. Straps 546 pass through openings in housing 540 and through hole 525 in main body 520 and are held in place by screw 547. Figure 5C also depicts a first direction 513, *i.e.* a general direction of light emitted by laser diodes 512 and LED's 514, 516, as well as a second direction 533, *i.e.* a general direction of light emitted by laser diodes 532 and LED's 534, 536. In the depicted embodiment, second surface 524 is angled by less than 20° relative to first surface 522, and second direction 533 is within 20° of opposite first direction 513 as measured in the depicted plane perpendicular to first surface 522. Furthermore, first surface 522 and second surface 524 are depicted as being angled such that first surface 522 and second surface 524 are closer together toward the viewer. Likewise, first window 541 and second window 542 are depicted as being angled such that first window 541 and second window 542 are closer together toward the viewer. First controller 515 controls a supply of power to laser diodes 512 and LED's 514, 516 in response to a signal from first detector 517, *e.g.* a signal indicative of whether a dairy animal is in an irradiation zone of laser diodes 512 and LED's 514, 516. Second controller 535 controls a supply of power to laser diodes 532 and LED's 534, 536 in response to a signal from first detector 537, *e.g.* a signal indicative of whether a dairy animal is in an irradiation zone of laser diodes 532 and LED's 534, 536.

Figure 5D shows a schematic depiction of a top side of the laser irradiation device 510 of Fig. 5A. In the depicted embodiment, laser irradiation device 510 is shown as comprising through holes 525, straps 527, and housing 540. As seen from above, laser irradiation device 510 exhibits a wedge-shaped profile as also portrayed in Figure 5C. Figure 5D also depicts first direction 513 and second direction 533. Although not antiparallel to first direction 513, second direction 533 is within 20° of opposite first direction 513 as measured in the depicted horizontal plane.

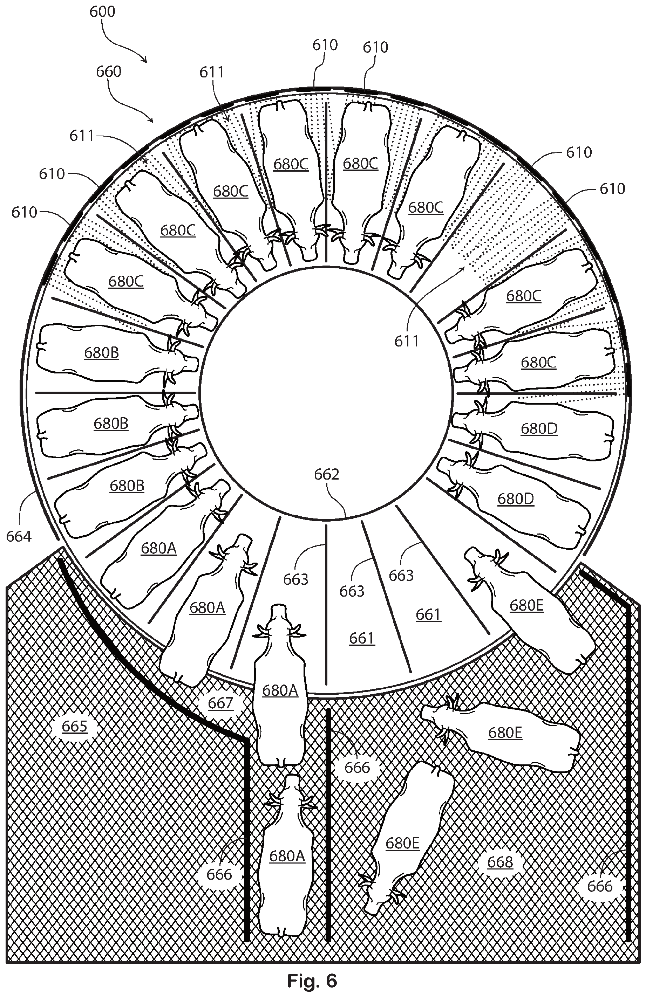

Figure 6 shows a schematic depiction of a milking system 600 in accordance with the present disclosure, e.g. as described above. In the depicted embodiment, milking system 600 comprises a plurality of irradiation devices 610, and a milking carousel 660. Milking carousel 660 comprises a plurality of milking stalls 661 that are defined by a central circular fence 662, an outer perimeter fence 664, and dividers 663. Dairy animals 680A-680E enter and exit milking carousel 660 from a platform 665 that is subdivided by corral fencing 666 into an entrance corral 667 and an exit corral 668. Specifically, dairy animals 680A enter milking carousel 660 from entrance corral 667. As milking carousel 660 rotates, dairy animals 680B are first prepared for milking by attaching milking cups of milking carousel 660 to teats of dairy animals 680B (not shown in Figure 6). During further rotation of milking carousel 660, dairy animals 680C are milked via the milking cups and irradiated with light 611 emitted from irradiation devices 610. After milking, the milking cups are removed from dairy animals 680D, and, upon reaching exit corral 668, dairy animals 680E exit milking carousel 660. The individual irradiation devices 610 are mounted on outer perimeter fence 664 of milking carousel 660 and irradiate light 611 toward a center of milking carousel 660, i.e. toward a rear surface of an udder of the respective, individual dairy animals 680C in milking stalls 661. In the depicted embodiment, irradiation devices 610 irradiate light 611 whenever milking carousel 660 is in operation, i.e. regardless of whether a dairy animal 680C is present in a stall passing through an irradiation zone of the respective irradiation devices 610.

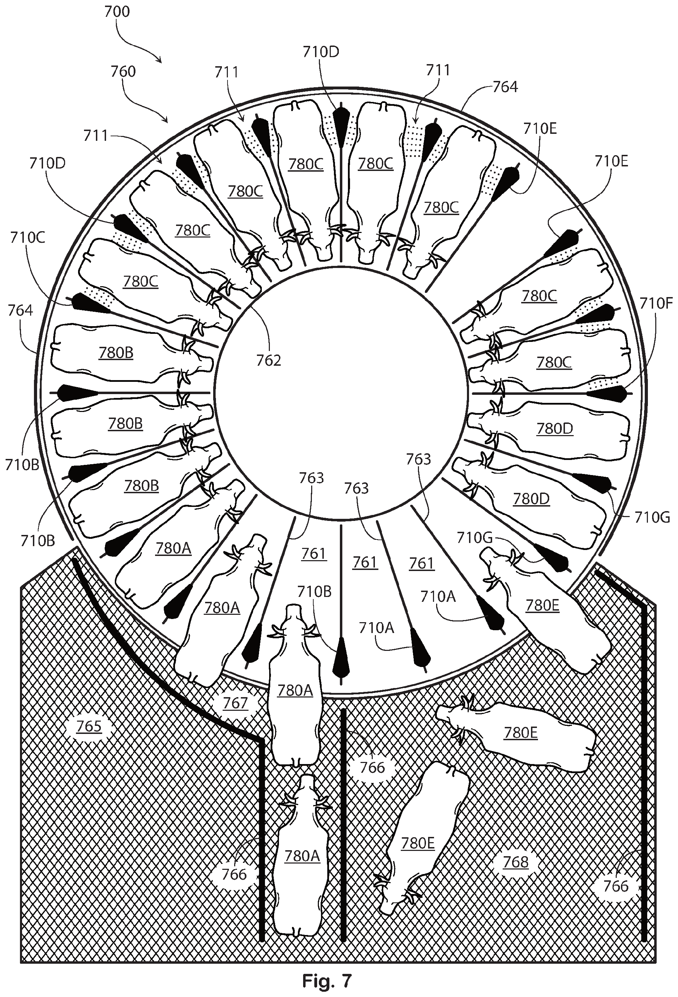

Figure 7 shows a schematic depiction of a milking system 700 in accordance with the present disclosure, *e.g.* as described above. In the depicted embodiment, milking system 700 comprises a plurality of irradiation devices 710A-710G, and a milking carousel 760. Milking carousel 760 comprises a plurality of milking stalls 761 that are defined by a central circular fence 762, an outer perimeter fence 764, and dividers 763. Dairy animals 780A-780E enter and exit milking carousel 760 from a platform 765 that is subdivided by corral fencing 766 into an entrance corral 767 and an exit corral 768. Specifically, dairy animals 780A enter milking carousel 760 from entrance corral 767. As milking carousel 760 rotates, dairy animals 780B are first prepared for milking by attaching milking cups of milking carousel 760 to teats of dairy animals 780B (not shown in Figure 7). During further rotation of milking carousel 760, dairy animals 780C are milked via the milking cups and irradiated with light 711 emitted from irradiation devices 710C-710F. After milking, the milking cups are removed from dairy animals 780D, and, upon reaching exit corral 768, dairy animals 780E exit milking carousel 760. In the depicted embodiment, irradiation devices 710A-710G are controlled as a function of time and the presence of a diary animal 780A-780E. In a first state, irradiation devices 710A do not emit light. Upon detection of a diary animal 780A in an associated irradiation zone, irradiation devices 710B transition to a second state, but do not emit light for a first amount of time. After elapse of the first amount of time, irradiation devices 710D transition to a third state and emit light for a second amount of time. During transition to the third state, irradiation devices 710C that irradiate distinct irradiation zones often irradiate only a subset of those irradiation zones, for example when dairy animals enter the respective irradiation zones at different times. In the third state, irradiation devices 710E that irradiate distinct irradiation zones occasionally also irradiate only a subset of those irradiation zones, for example when no dairy animal is present in a respective irradiation zone. After elapse of the second amount of time, irradiation devices 710G transition to a fourth state and cease to emit light. During transition to the fourth state, irradiation devices 710F that irradiate distinct irradiation zones often continue to irradiate a subset of those irradiation zones, for example when dairy animals entered the respective irradiation zones at different times. Upon detection of an absence of a diary animal 780A in an associated irradiation zone, irradiation devices 710A transition to the first state. If a dairy animal does not leave stall 761 as stall 761 passes exit corral 768, irradiation devices adjacent the respective stall 761 at least partially remain in the first state and do not irradiate the respective stall 761 until the dairy animal leaves the respective stall 761.

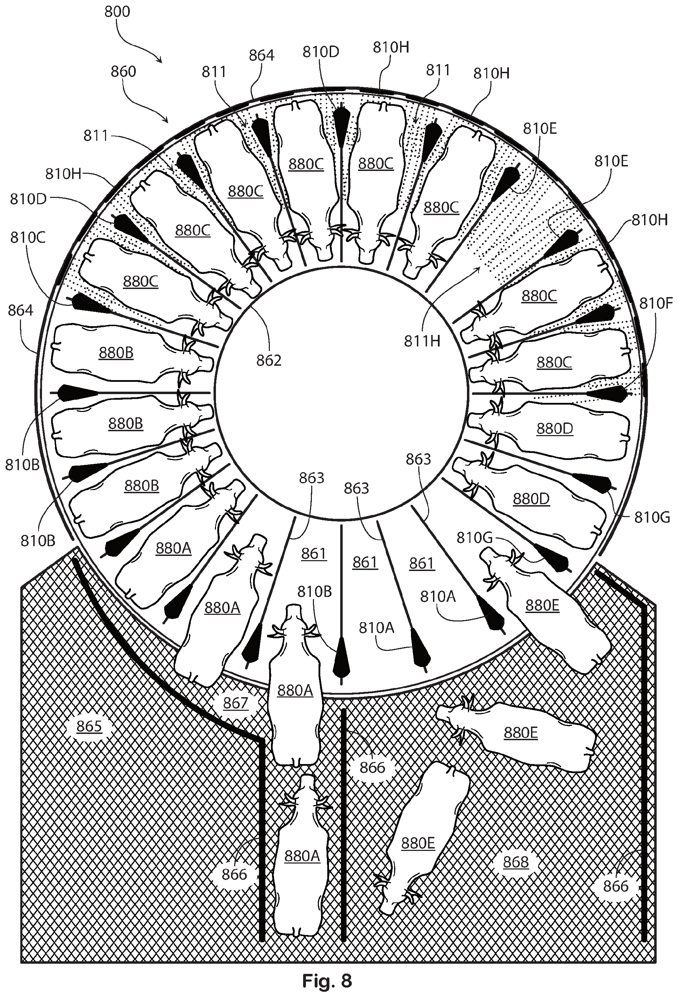

Figure 8 shows a schematic depiction of a milking system 800 in accordance with the present disclosure, *e.g.* as described above. In the depicted embodiment, milking system 800 comprises a plurality of irradiation devices 810A-810H, and a milking carousel 860. Milking carousel 860 comprises a plurality of milking stalls 861 that are defined by a central circular fence 862, an outer perimeter fence 864, and dividers 863. Dairy animals 880A-880E enter and exit milking carousel 860 from a platform 865 that is subdivided by corral fencing 866 into an entrance corral 867 and an exit corral 868. Specifically, dairy animals 880A enter milking carousel 860 from entrance corral 867. As milking carousel 860 rotates, dairy animals 880B are first prepared for milking by attaching milking cups of milking carousel 860 to teats of dairy animals 880B (not shown in Figure 8). During further rotation of milking carousel 860, dairy animals 880C are milked via the milking cups and irradiated with light 811 emitted from irradiation devices 810C-810F, 810H. After milking, the milking cups are removed from dairy animals 880D, and, upon reaching exit corral 868, dairy animals 880E exit milking carousel 860. A plurality of individual irradiation devices 810H is mounted on outer perimeter fence 864 of milking carousel 860 and irradiate light 811H toward a center of milking carousel 860, *i.e.* toward a rear surface of an udder of the respective, individual dairy animals 880C in milking stalls 861. In the depicted embodiment, irradiation devices 810H irradiate light 811H whenever milking carousel 860 is in operation, *i.e.* regardless of whether a dairy animal 880C is present in a stall passing through an irradiation zone of the respective irradiation devices 810H. In contrast, irradiation devices 810A-810G are controlled as a function of time and the presence of a diary animal 880A-880E. In a first state, irradiation devices 810A do not emit light. Upon detection of a diary animal 880A in an associated irradiation zone, irradiation devices 810B transition to a second state, but do not emit light for a first amount of time. After elapse of the first amount of time, irradiation devices 810D transition to a third state and emit light for a second amount of time. During transition to the third state, irradiation devices 810C that irradiate distinct irradiation zones often irradiate only a subset of those irradiation zones, for example when dairy animals enter the respective irradiation zones at different times. In the third state, irradiation devices 810E that irradiate distinct irradiation zones occasionally also irradiate only a subset of those irradiation zones, for example when no dairy animal is present in a respective irradiation zone. After elapse of the second amount of time, irradiation devices 810G transition to a fourth state and cease to emit light. During transition to the fourth state, irradiation devices 810F that irradiate distinct irradiation zones often continue to irradiate a subset of those irradiation zones, for example when dairy animals entered the respective irradiation zones at different times. Upon detection of an absence of a diary animal 880A in an associated irradiation zone, irradiation devices 810A transition to the first state. If a dairy animal does not leave stall 861 as stall 861 passes exit corral 868, irradiation devices adjacent the respective stall 861 at least partially remain in the first state and do not irradiate the respective stall 861 until the dairy animal leaves the respective stall 861.

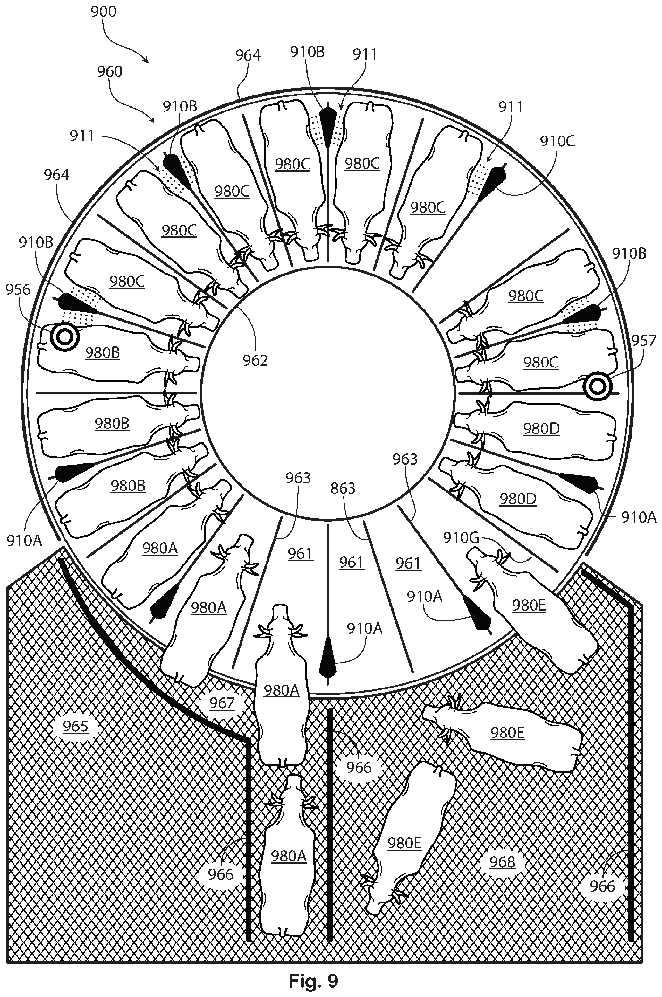

Figure 9 shows a schematic depiction of a milking system 900 in accordance with the present disclosure, *e.g.* as described above. In the depicted embodiment, milking system 900 comprises a plurality of irradiation devices 910A-910C, and a milking carousel 960. Milking carousel 960 comprises a plurality of milking stalls 961 that are defined by a central circular fence 962, an outer perimeter fence 964, and dividers 963. In the depicted embodiment, only one irradiation device 910A-910C is provided per stall, thus limiting irradiation to only one side of an udder of dairy animal 980A-980E per milking cycle. Dairy animals 980A-980E enter and exit milking carousel 9960 from a platform 965 that is subdivided by corral fencing 966 into an entrance corral 967 and an exit corral 968. Specifically, dairy animals 980A enter milking carousel 960 from entrance corral 967. As milking carousel 960 rotates, dairy animals 980B are first prepared for milking by attaching milking cups of milking carousel 960 to teats of dairy animals 980B (not shown in Figure 9). During further rotation of milking carousel 960, dairy animals 980C are milked via the milking cups and irradiated with light 911 emitted from irradiation devices 910B, 910C. After milking, the milking cups are removed from dairy animals 980D, and, upon reaching exit corral 968, dairy animals 980E exit milking carousel 960. In the depicted embodiment, irradiation devices 910A-910C are controlled as a function of location and the presence of a diary animal 980A-980E. In a first state, irradiation devices 910A do not emit light. Upon detection of a first magnetic location 956, irradiation devices 910B, 910C transition to a second state. In the second state, irradiation devices 910B, 910C emit light as a function of the presence of a diary animal 980A-9780E. Specifically, as exemplified by irradiation device 910C, irradiation devices 910B, 910C in the second state irradiate only those irradiation zones where a dairy animal is detected as present. Upon detection of a second magnetic location 957, irradiation devices 910A transition back to the first state and cease to emit light. The location of first magnetic location 956 and second magnetic location 957 is programmed into irradiation devices 910A-910C individually by invoking an appropriate command via a wired or wireless interface when the individual irradiation device 910A-910C is situated at the first / second magnetic location 956, 957 to store data obtained from a respective magnetic position sensor. The individual irradiation devices 910A-910C later compare the stored data with current data obtained from the respective magnetic position sensor to detect the first magnetic location 956 and the second magnetic location 957 as carousel 960 rotates.

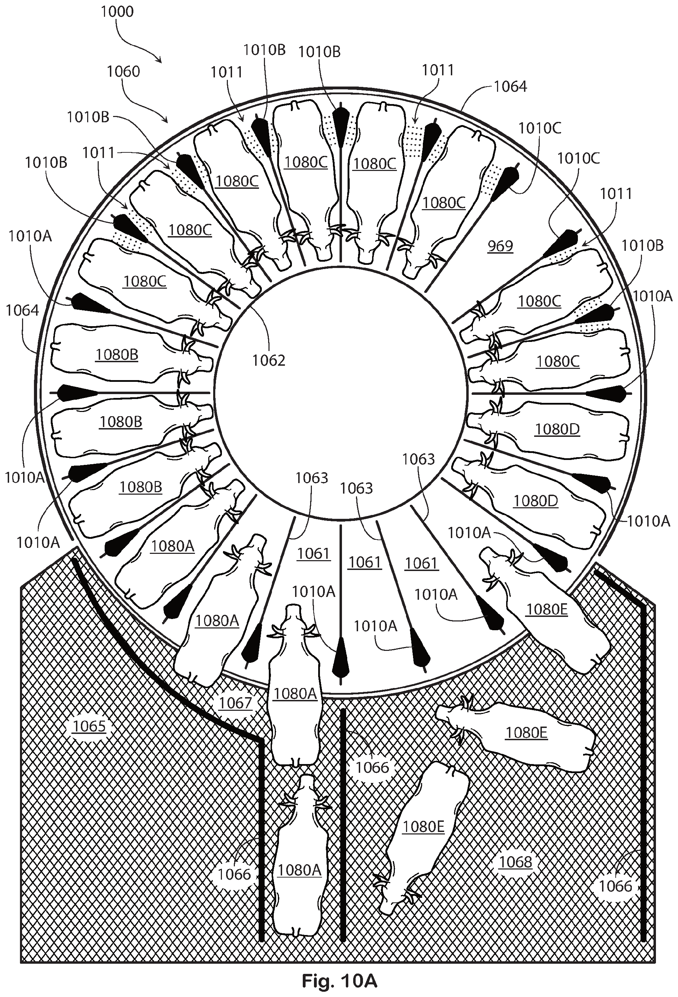

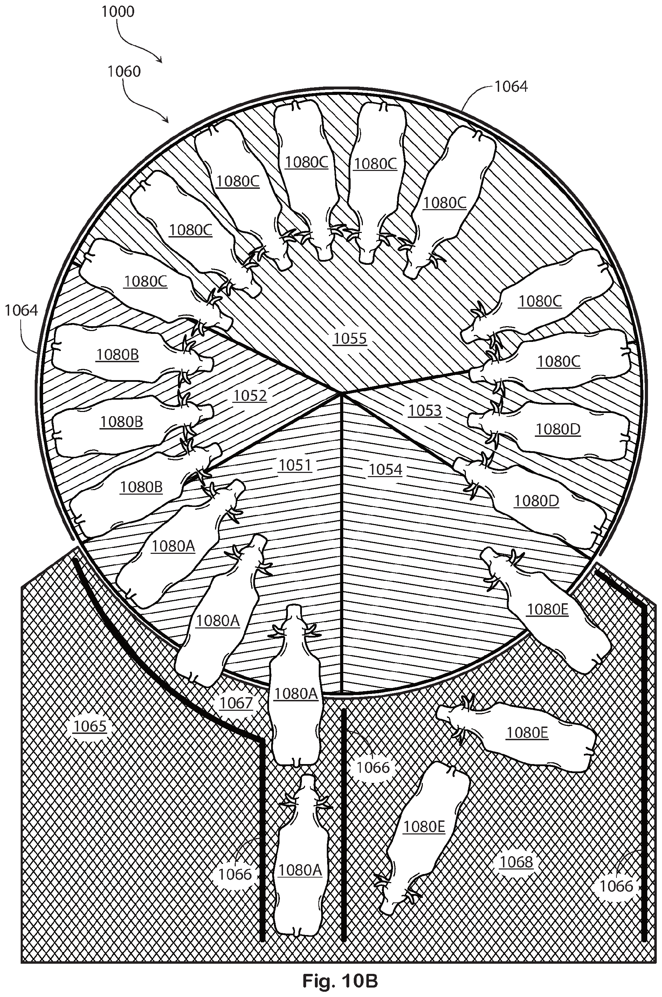

Figure 10A shows a first schematic depiction of a milking system 1000 in accordance with the present disclosure, *e.g.* as described above. In the depicted embodiment, milking system 1000 comprises a plurality of irradiation devices 1010A-1010C, and a milking carousel 1060. Milking carousel 1060 comprises a plurality of milking stalls 1061 that are defined by a central circular fence 1062, an outer perimeter fence 1064, and dividers 1063. Dairy animals 1080A-1080E enter and exit milking carousel 1060 from a platform 1065 that is subdivided by corral fencing 1066 into an entrance corral 1067 and an exit corral 1068. Specifically, dairy animals 1080A enter milking carousel 1060 from entrance corral 1067. As milking carousel 1060 rotates, dairy animals 1080B are first prepared for milking by attaching milking cups of milking carousel 1060 to teats of dairy animals 1080B (not shown in Figure 10). During further rotation of milking carousel 1060, dairy animals 1080C are milked via the milking cups and irradiated with light 1011 emitted from irradiation devices 1010B, 1010C. After milking, the milking cups are removed from dairy animals 1080D, and, upon reaching exit corral 1068, dairy animals 1080E exit milking carousel 1060. **In** the depicted embodiment, operation of the individual irradiation devices 1010A-1010C is effected as a function of an angular position of milking carousel 1060 and the presence of a diary animal 1080A-10780E in an associated irradiation zone. More specifically, irradiation devices 1010A temporarily located, as milking carousel 1060 rotates, in any of the circular sectors where dairy animals 1080A enter milking carousel 1060, where dairy animals 1080B are prepared for milking, where milking cups are removed from dairy animals 1080D, or where dairy animals 1080E exit milking carousel 1060 are disconnected from power, *e.g.* by actuation of respective switches as milking carousel 1060 rotates. Conversely, irradiation devices 1010B, 1010C temporarily not located in any of the aforementioned circular sectors are supplied with electrical power and are capable of irradiating both adjacent stalls with light 1011. Where a dairy animal 1080C is present in each adjacent stall, individual irradiation devices 910B irradiate both adjacent stalls. Where a dairy animal 1080C is present in only one of the two adjacent stalls, individual irradiation devices 1010C do not irradiate the empty stall 1069 where no dairy animal is present.

Figure 10B shows a second schematic depiction of milking system 1000 of Figure 10A. In particular, Figure 10B shows a subdivision of a circle 1050 defined by a perimeter of milking carousel 1060 of milking system 1000 into a set of five non-overlapping circular sectors. Specifically, it shows a first circular sector 1051 corresponding to a section of milking carousel 1060 adjacent entrance corral 1067. A second circular sector 1052 corresponds to a section of milking carousel 1060 where dairy animals 1080B are prepared for milking, e.g. by attaching milking cups to the teats of dairy animals 1080B. A third circular sector 1053 corresponds to a section of milking carousel 1060 where the milking cups are removed from dairy animals 1080D. The set may comprise a fourth circular sector 1054 corresponding to a section of milking carousel 1060 adjacent the exit corral. The set may comprise a fifth circular sector 1055 corresponding to a section of milking carousel 1060 where dairy animals 1080C undergo milking. As in Figure 10A, platform 1065 is subdivided by corral fencing 1066 into entrance corral 1067 and exit corral 1068, and outer perimeter fence 1064 occludes a portion of a circumference of milking carousel 1060.

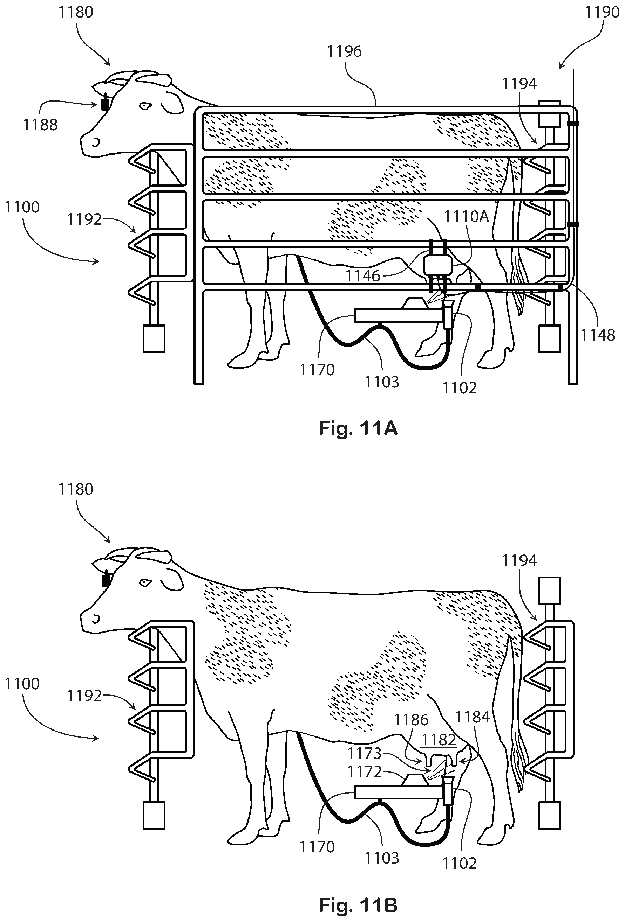

Figure 11A shows a first schematic depiction of a milking system 1100 in accordance with the present disclosure, e.g. as described above. **In** the depicted embodiment, milking system 1100 is shown as comprising a first milking cup 1102, a first hose 1103, a first laser irradiation device 1110A, and a robotic arm 1170. In the depicted embodiment, a dairy animal 1180 is confined in a milking stall 1190 that comprises a front gate 1192 that restricts a position of dairy animal 1180 in a forward direction, a rear gate 1194 that restricts a position of dairy animal 1180 in a rearward direction, and side fencing 1196 that restricts a position of dairy animal 1180 in a lateral direction. Dairy animal 1180 is tagged with an RFID tag 1188 for identification. First laser irradiation device 1110A is hung at an appropriate height to irradiate an udder of dairy animal 1180 by straps 1146 between respective tubes of side fencing 1196. A power cord 1148 of first laser irradiation device 1110A is affixed to side fencing 1196 to prevent entanglement with dairy animal 1180.

Figure 11B shows a second schematic depiction of milking system 1100 of Figure 11A. Milking system 1100 is again shown as comprising first milking cup 1102, first hose 1103, and robotic arm 1170. In the depicted embodiment, robotic arm 1170 is shown as comprising an optical teat detector 1172 that emits a scanning beam of light 1173 to detect a position of a first teat 1184 and a second teat 1186 of an udder 1182 of dairy animal 1180 confined, *inter alia,* by front gate 1192 and rear gate 1194.

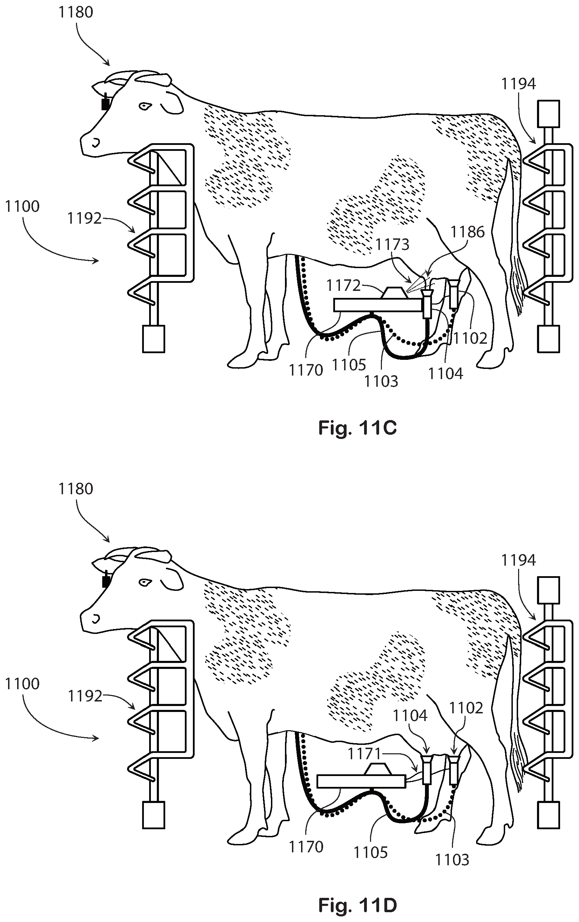

Figure 11C shows a third schematic depiction of milking system 1100 of Figure 11A. **In** the depicted embodiment, milking system 1100 is shown as comprising a second milking cup 1104 and a second hose 1105 in addition to first milking cup 1102, first hose 1103, and robotic arm 1170. Optical teat detector 1172 is shown as emitting a scanning beam of light 1173 to detect a position of second teat 1186 of dairy animal 1180 confined, *inter alia,* by front gate 1192 and rear gate 1194. First milking cup 1102 is depicted as being attached to the first teat (not visible in the depiction) of dairy animal 1180 for the sake of milking.

Figure 11D shows a fourth schematic depiction of milking system 1100 of Figure 10A. In the depicted embodiment, milking system 1100 is again shown as comprising first milking cup 1102, first hose 1103, second milking cup 1104, second hose 1105, and robotic arm 1170. Robotic arm 1170 comprises tie strings 1171 that prevent first milking cup 1102 and second milking cup 1104 from falling to the ground and permit retraction of first milking cup 1102 and second milking cup 1104 to robotic arm 1170. Dairy animal 1180 is depicted as being confined, *inter alia,* by front gate 1192 and rear gate 1194. First milking cup 1102 and second milking cup 1104 are depicted as being attached to the first teat and the second teat (both not visible in the depiction), respectively, of dairy animal 1180 for the sake of milking.

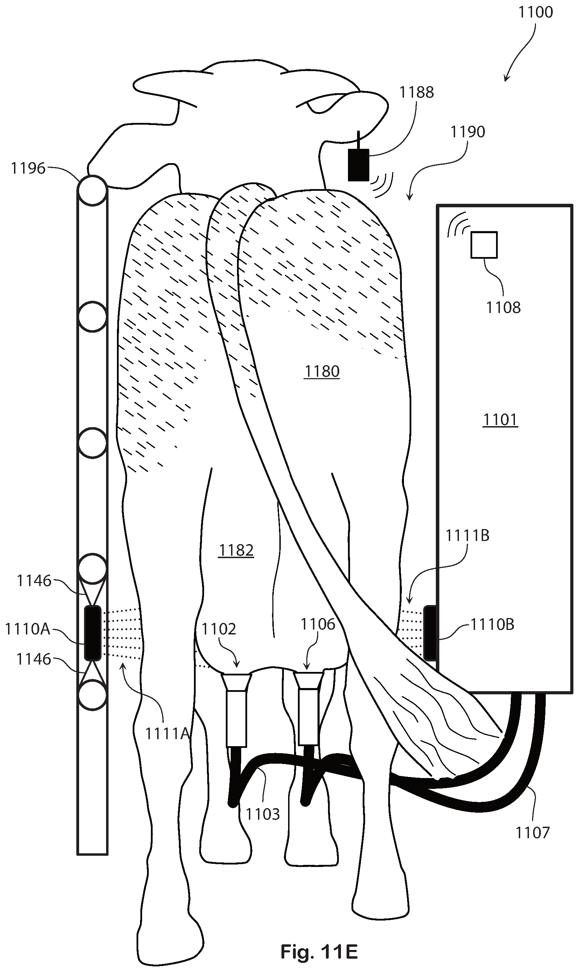

Figure 11E shows a fifth schematic depiction of milking system 1100 of Figure 11A. In the depicted embodiment, milking system 1100 is shown as comprising a milking controller 1101, a third milking cup 1106, a third hose 1107, and a second irradiation device 1110B in addition to first milking cup 1102, first hose 1103, and first laser irradiation device 1110A. First laser irradiation device 1110A is hung by straps 1146 at an appropriate height to irradiate light 1111A onto a left surface of udder 1182. Second laser irradiation device 1110A is attached to milking controller 1101 at an appropriate height at an appropriate height to irradiate light 1111B onto a right surface of udder 1182. Milking controller 1101 comprises an RFID reader 1108 that communicates with RFID tag 1188 to detect the presence of dairy animal 1180 in a milking stall 1190 defined, *inter alia*, by side fencing 1196 and milking controller 1101 that restrict a position of dairy animal 1180 in a lateral direction. First milking cup 1102 and third milking cup 1106 are depicted as being attached to the first teat and a third teat (both not visible in the depiction), respectively, of dairy animal 1180 for the sake of milking.

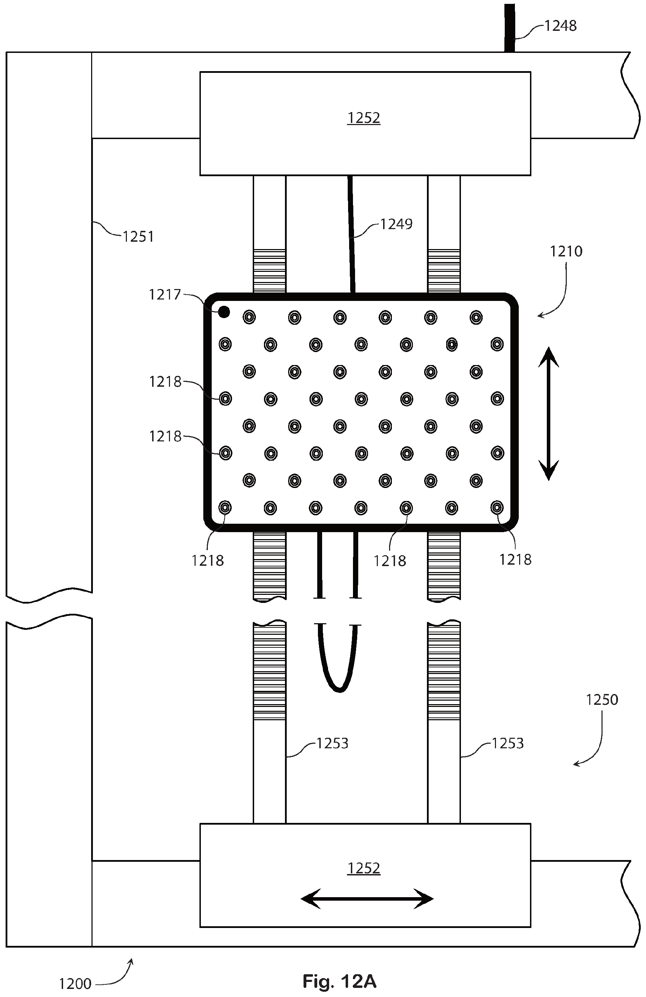

Figure 12A shows a first schematic depiction of an irradiation system 1200 in accordance with the present disclosure, *e.g.* as described above. In the depicted embodiment, irradiation system 1200 comprises an irradiation device 1210, a positioning system 1250, a power cord 1248, and a cable 1249. Irradiation device 1210 is shown as comprising a detector 1217 and a plurality of focussed SLE's 1218. Positioning system 1250 is shown as comprising a frame 1251 and two carriages 1252 that glide on frame 1251 to move irradiation device 1210 in a horizontal direction. Positioning system 1250 also comprises two toothed rods 1253 that are mounted between carriages 1252 and allow a controlled motion of irradiation device 1210 in a vertical direction.

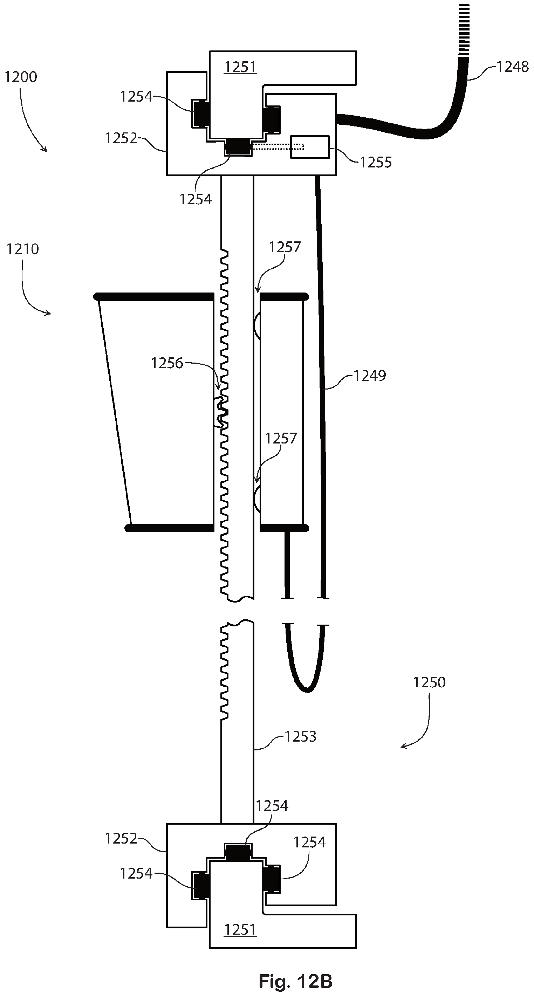

Figure 12B shows a second schematic depiction of irradiation system 1200 of Figure 12A. In the depicted embodiment, irradiation system 1200 comprises irradiation device 1210, positioning system 1250, power cord 1248, and cable 1249. Positioning system 1250 is shown as comprising frame 1251, carriages 1252, and toothed rod 1253 that are mounted between carriages 1252. Carriages 1252 comprise rollers 1254 that roll on corresponding surfaces of frame 1251, including a roller 1254 driven by a motor 1255 under the control of a controller (not shown), *e.g.* using signals from detector 1217 as described above. Irradiation device 1210 is shown as comprising a cog wheel 1256 and two rollers 1257 that allow a controlled motion of irradiation device 1210 in a vertical direction. Cog wheel is driven by a motor (not shown) under the control of a controller (not shown), *e.g.* using signals from detector 1217 as described above to direct light emitted by focussed SLE's 1218 onto an udder.

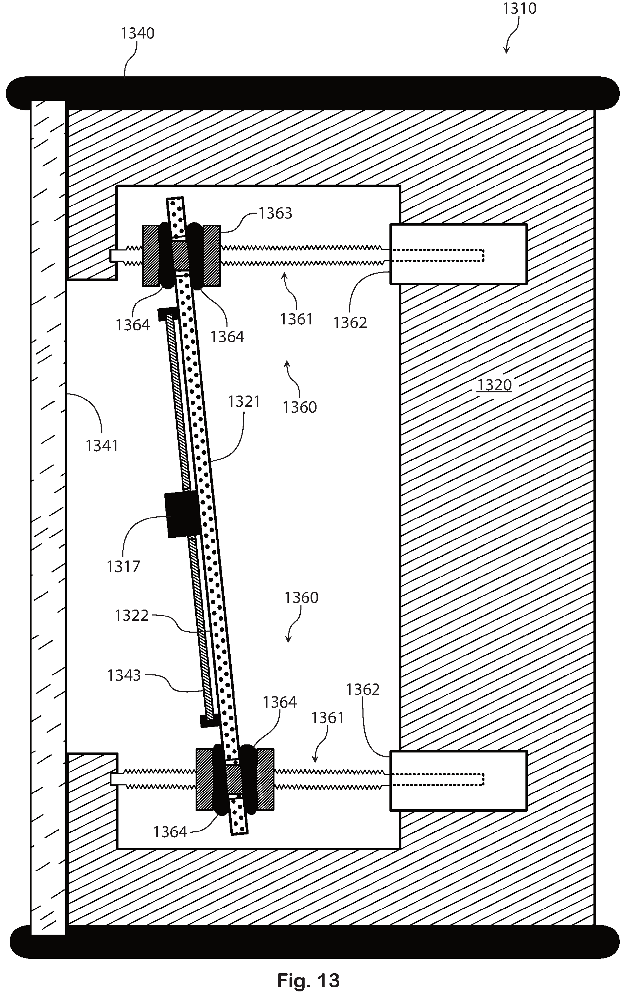

Figure 13 shows a first schematic depiction of an irradiation device 1310 in accordance with the present disclosure, *e.g.* as described above. In the depicted embodiment, irradiation device 1310 comprises a detector 1317, a main body 1320, a printed circuit board 1321, a housing 1340, a window 1341, a lens carrier 1343, and a positioning system 1360. Detector 1317 is mounted on a first surface 1322 of printed circuit board 1321 that supports a plurality of SLE's (not shown). Lens carrier 1343 focuses light emitted from the SLE'S on first surface 1322. Positioning system 1360 comprises four spindles 1361, four motors 1362, four threaded connectors 1363, and elastic washers 1364 that elastically support printed circuit board 1321 *vis-à-vis* threaded connectors 1363. Only two of the four spindles 1361, motors 1362, and threaded connectors 1363 are shown. Spindles 1361 are driven by motor 1362 under the control of a controller (not shown), e.g. using signals from detector 1317 as described above to alter an orientation of printed circuit board 1321 in two dimensions and direct light emitted by the SLE's onto an udder.

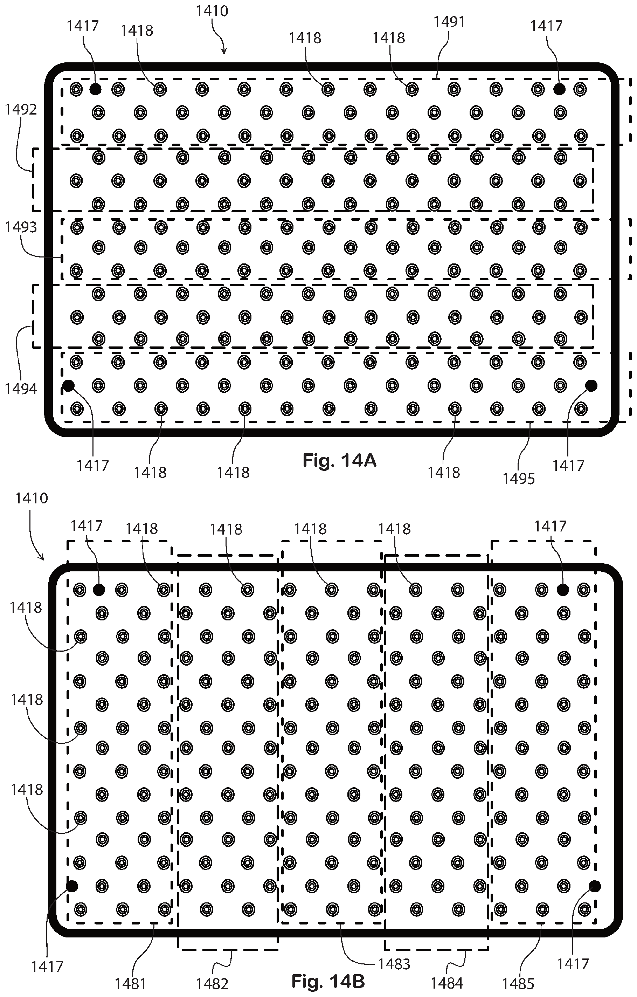

Figure 14A shows a first schematic depiction of an irradiation device 1410 in accordance with the present disclosure, e.g. as described above. In the depicted embodiment, irradiation device 1310 comprises detectors 1417 and a plurality of focussed SLE's 1418. The plurality of focussed SLE's 1418 is divided into five subsets of focussed SLE's 1418, namely a first subset of focussed SLE's 1418 situated in a first vertical zone 1481, a second subset of focussed SLE's 1418 situated in a second vertical zone 1482, a third subset of focussed SLE's 1418 situated in a third vertical zone 1483, a fourth subset of focussed SLE's 1418 situated in a fourth vertical zone 1484, and a fifth subset of focussed SLE's 1418 situated in a fifth vertical zone 1485. A controller (not shown) interrupts a supply of power to a respective subset in response to a signal from detectors 1417 indicative of an absence of an udder in an irradiation zone of the respective subset.

Figure 14B shows a second schematic depiction of irradiation device 1410 of Figure 14A. **In** the depicted embodiment, the plurality of focussed SLE's 1418 is divided into five subsets of focussed SLE's 1418, namely a sixth subset of focussed SLE's 1418 situated in a first horizontal zone 1481, a seventh subset of focussed SLE's 1418 situated in a second horizontal zone 1482, an eighth subset of focussed SLE's 1418 situated in a third horizontal zone 1483, a ninth subset of focussed SLE's 1418 situated in a fourth horizontal zone 1484, and a tenth subset of focussed SLE's 1418 situated in a fifth horizontal zone 1485. A controller (not shown) interrupts a supply of power to a respective subset in response to a signal from detectors 1417 indicative of an absence of an udder in an irradiation zone of the respective subset.

In the present disclosure, the verb "may" is used to designate optionality / noncompulsoriness. In other words, something that "may" can, but need not. In the present disclosure, the verb "comprise" may be understood in the sense of including. Accordingly, the verb "comprise" does not exclude the presence of other elements / actions. **In** the present disclosure, relational terms such as "first," "second," "top," "bottom" and the like may be used solely to distinguish one entity or action from another entity or action without necessarily requiring or implying any actual such relationship or order between such entities or actions.

In the present disclosure, the term "any" may be understood as designating any number of the respective elements, *e.g.* as designating one, at least one, at least two, each or all of the respective elements. Similarly, the term "any" may be understood as designating any collection(s) of the respective elements, *e.g.* as designating one or more collections of the respective elements, wherein a (respective) collection may comprise one, at least one, at least two, each or all of the respective elements. The respective collections need not comprise the same number of elements.

In the present disclosure, the expression "at least one" is used to designate any (integer) number or range of (integer) numbers (that is technically reasonable in the given context). As such, the expression "at least one" may, *inter alia*, be understood as one, two, three, four, five, ten, fifteen, twenty or one hundred. Similarly, the expression "at least one" may, *inter alia*, be understood as "one or more," "two or more" or "five or more."

In the present disclosure, expressions in parentheses may be understood as being optional. As used in the present disclosure, quotation marks may emphasize that the expression in quotation marks may also be understood in a figurative sense. As used in the present disclosure, quotation marks may identify a particular expression under discussion.

In the present disclosure, many features are described as being optional, *e.g.* through the use of the verb "may" or the use of parentheses. For the sake of brevity and legibility, the present disclosure does not explicitly recite each and every combination and/or permutation that may be obtained by choosing from the set of optional features. However, the present disclosure is to be interpreted as explicitly disclosing all such combinations / permutations. For example, a system described as having three optional features may be embodied in seven different ways, namely with just one of the three possible features, with any two of the three possible features or with all three of the three possible features.

While various embodiments of the present invention have been disclosed and described in detail herein, it will be apparent to those skilled in the art that various changes may be made to the configuration, operation and form of the invention without departing from the spirit and scope thereof. In particular, it is noted that the respective features of the invention, even those disclosed solely in combination with other features of the invention, may be combined in any configuration excepting those readily apparent to the person skilled in the art as nonsensical. Likewise, use of the singular and plural is solely for the sake of illustration and is not to be interpreted as limiting. Except where the contrary is explicitly noted, the plural may be replaced by the singular and vice-versa.

The above disclosure may be summarized as comprising the following embodiments.

### Embodiment 1:

A (laser) irradiation device, comprising:
a first plurality of semiconductor light emitters / laser diodes; and
a support, wherein
said support comprises a first planar surface,
said first plurality of semiconductor light emitters / laser diodes are distributed on said first planar surface,
each semiconductor light emitter / laser diode of said first plurality of semiconductor light emitters / laser diodes emits light at a wavelength greater than 800 nm and less than 1100 nm,
said first planar surface is at least 10 cm times 10 cm in area, and
said first plurality of semiconductor light emitters / laser diodes is structured to irradiate an area of at least 15 cm times 15 cm in a first imaginary plane parallel to and 50 cm from said first planar surface.

### Embodiment 2:

The (laser) irradiation device of Embodiment 1, wherein:
said first plurality of semiconductor light emitters / laser diodes irradiates said area in said first imaginary plane such that an average irradiation intensity in said area is not less than 40% of a maximum irradiation intensity in said area.

### Embodiment 3:

The (laser) irradiation device of Embodiment 1 or 2, comprising:
a second plurality of semiconductor light emitters / light-emitting diodes, wherein
said second plurality of semiconductor light emitters / light-emitting diodes are distributed on said first planar surface,
each semiconductor light emitter / light-emitting diode of said second plurality of semiconductor light emitters / light-emitting diodes emits light at a wavelength greater than 400 nm and less than 800 nm, and
said second plurality of semiconductor light emitters / light-emitting diodes is structured to irradiate said area of at least 15 cm times 15 cm in said first imaginary plane.

### Embodiment 4:

The (laser) irradiation device of Embodiment 3, wherein:
said second plurality of semiconductor light emitters / light-emitting diodes irradiates said area in said first imaginary plane such that an average irradiation intensity effected by said second plurality of semiconductor light emitters / light-emitting diodes in said area is not less than 40% of a maximum irradiation intensity effected by said second plurality of semiconductor light emitters / light-emitting diodes in said area.

### Embodiment 5:

The (laser) irradiation device of any one of the preceding Embodiments, comprising:
a power supply system that supplies electrical power to said first plurality of semiconductor light emitters / laser diodes, wherein
said laser irradiation device is structured to protect said power supply system and said first plurality of semiconductor light emitters / laser diodes against an ingress of liquid at a rating of at least IPX5 according to international standard IEC 60529.

### Embodiment 6:

The (laser) irradiation device of any one of the preceding Embodiments, comprising:
a detector structured to distinguish between a presence and an absence of an irradiation target, and
a controller structured to interrupt a supply of power to said first plurality of semiconductor light emitters / laser diodes in response to a signal from said detector indicative of an absence of an irradiation target.

### Embodiment 7:

The (laser) irradiation device of any one of the preceding Embodiments, comprising:
a second plurality of semiconductor light emitters / laser diodes, wherein
said support comprises a second planar surface,
said second plurality of semiconductor light emitters / laser diodes are distributed on said second planar surface,
each semiconductor light emitter / laser diode of said second plurality of semiconductor light emitters / laser diodes emits light at a wavelength greater than 800 nm and less than 1100 nm,
each semiconductor light emitter / laser diode of said first plurality of semiconductor light emitters / laser diodes emits light in a first direction,
each semiconductor light emitter / laser diode of said second plurality of semiconductor light emitters / laser diodes emits light in a second direction not more than 30° from opposite said first direction,
said second planar surface is at least 10 cm times 10 cm in area, and
said second plurality of semiconductor light emitters / laser diodes is structured to irradiate an area of at least 15 cm times 15 cm in a second imaginary plane parallel to and 50 cm from said second planar surface.

### Embodiment 8:

A milking system, comprising:
a first milking cup structured to receive a first teat of an udder;
a second milking cup structured to receive a second teat of said udder; and
a (laser) irradiation device structured to bathe a surface of said udder in light at a wavelength greater than 400 nm and less than 1100 nm, wherein
said light-bathed udder surface has an area of at least 100 cm².

### Embodiment 9:

The milking system of Embodiment 8, wherein:
said (laser) irradiation device comprises:
   a first plurality of semiconductor light emitters / laser diodes; and
   a support,
said support comprises a first planar surface,
said first plurality of semiconductor light emitters / laser diodes are distributed on said first planar surface,
each semiconductor light emitter / laser diode of said first plurality of semiconductor light emitters / laser diodes emits light at a wavelength greater than 800 nm and less than 1100 nm,
said first planar surface is at least 10 cm times 10 cm in area, and
said first plurality of semiconductor light emitters / laser diodes is structured to irradiate an area of at least 15 cm times 15 cm in a first imaginary plane parallel to and 50 cm from said first planar surface.

Embodiment 10:

The milking system of Embodiment 9, wherein:
said first plurality of semiconductor light emitters / laser diodes irradiates said area in said first imaginary plane such that an average irradiation intensity in said area is not less than 40% of a maximum irradiation intensity in said area.

### Embodiment 11:

The milking system of Embodiment 9 or 10, comprising:
a plurality of light-emitting diodes, wherein
said plurality of light-emitting diodes are distributed on said first planar surface,
each light-emitting diode of said plurality of light-emitting diodes emits light at a wavelength greater than 400 nm and less than 800 nm, and
said plurality of light-emitting diodes is structured to irradiate said area of at least 15 cm times 15 cm in said first imaginary plane.

### Embodiment 12:

The milking system of Embodiment 11, wherein:
said plurality of light-emitting diodes irradiates said area in said first imaginary plane such that an average irradiation intensity effected by said plurality of light-emitting diodes in said area is not less than 40% of a maximum irradiation intensity effected by said plurality of light-emitting diodes in said area.

### Embodiment 13:

The milking system of any one of Embodiments 9-11, wherein:
said (laser) irradiation device comprises a power supply system that supplies electrical power to said first plurality of semiconductor light emitters / laser diodes, and
said (laser) irradiation device is structured to protect said power supply system and said first plurality of semiconductor light emitters / laser diodes against an ingress of liquid at a rating of at least IPX5 according to international standard IEC 60529.

### Embodiment 14:

The milking system of any one of Embodiments 9-13, comprising:
a detector structured to distinguish between a presence and an absence of an irradiation target, and
a controller structured to interrupt a supply of power to said first plurality of semiconductor light emitters / laser diodes in response to a signal from said detector indicative of an absence of an irradiation target.

### Embodiment 15:

The milking system of any one of Embodiments 9-14, comprising:
a second plurality of semiconductor light emitters / laser diodes, wherein
said support comprises a second planar surface,
said second plurality of semiconductor light emitters / laser diodes are distributed on said second planar surface,
each laser diode of said second plurality of semiconductor light emitters / laser diodes emits light at a wavelength greater than 800 nm and less than 1100 nm,
each semiconductor light emitter / laser diode of said first plurality of semiconductor light emitters / laser diodes emits light in a first direction,
each semiconductor light emitter / laser diode of said second plurality of semiconductor light emitters / laser diodes emits light in a second direction not more than 30° from opposite said first direction,
said second planar surface is at least 10 cm times 10 cm in area, and
said second plurality of semiconductor light emitters / laser diodes is structured to irradiate an area of at least 15 cm times 15 cm in a second imaginary plane parallel to and 50 cm from said second planar surface.

### Embodiment 16:

A method, comprising:
equipping a milking system with a (laser) irradiation device, wherein
said milking system comprises a first milking cup structured to receive a first teat of an udder,
said milking system comprises a second milking cup structured to receive a second teat of said udder,
said (laser) irradiation device is structured to bathe a surface of said udder in light at a wavelength greater than 400 nm and less than 1100 nm, and
said light-bathed udder surface has an area of at least 100 cm².

### Embodiment 17:

The method of Embodiment 16, wherein:
said (laser) irradiation device comprises:
   a first plurality of semiconductor light emitters / laser diodes; and
   a support,
said support comprises a first planar surface,
said first plurality of semiconductor light emitters / laser diodes are distributed on said first planar surface,
each laser diode of said first plurality of semiconductor light emitters / laser diodes emits light at a wavelength greater than 800 nm and less than 1100 nm,
said first planar surface is at least 10 cm times 10 cm in area, and
said first plurality of semiconductor light emitters / laser diodes is structured to irradiate an area of at least 15 cm times 15 cm in a first imaginary plane parallel to and 50 cm from said first planar surface.

### Embodiment 18:

A method, comprising:
using a (laser) irradiation device to bathe a surface of an udder in light at a wavelength greater than 400 nm and less than 1100 nm, wherein
said light-bathed udder surface has an area of at least 100 cm².

### Embodiment 19:

The method of Embodiment 18, comprising:
milking said udder with a milking system, wherein
said milking system comprises a first milking cup structured to receive a first teat of an udder,
said milking system comprises a second milking cup structured to receive a second teat of said udder, and
said (laser) irradiation device bathes said udder surface in light during said milking.

### Embodiment 20:

A milking carousel, comprising:
an irradiation device, wherein
said irradiation device comprises a magnetic position sensor,
said irradiation device, using an output of said magnetic position sensor, switches to a light-emitting state at a first location during a rotation of said milking carousel, and
said irradiation device, using an output of said magnetic position sensor, switches to a non-light-emitting state at a second location during said rotation of said milking carousel.

### Embodiment 21:

A method, comprising:
switching an irradiation device mounted on a milking carousel to a light-emitting state at a first location during rotation of said milking carousel, and
switching said irradiation device to a non-light-emitting state at a second location during rotation of said milking carousel.

### Embodiment 22:

The method of Embodiment 21, wherein:
said switching to a light-emitting state is effected **in** response to an output of a magnetic position sensor.

### Embodiment 23:

A milking system, comprising:
a milking cup attachable to a teat of an udder;
a milk tester in fluid connection with said milking cup; and
an irradiation device structured to bathe a surface of said udder in light at a wavelength greater than 400 nm and less than 1100 nm, wherein
said milk tester measures parameters of milk milked from said teat,
a spectrum of light emitted by said irradiation device onto said udder is controlled as a function of said measured parameters.

### Embodiment 24:

A method, comprising:
milking milk from a teat of an udder using a milking cup,
measuring parameters of said milk using a milk tester in fluid connection with said milking cup, and
using an irradiation device to bathe a surface of said udder in light at a wavelength greater than 400 nm and less than 1100 nm, and
adjusting a spectrum of said light as a function of said measured parameters.

### Embodiment 25:

A laser irradiation device, comprising:
a plurality of laser diodes; and
a controller, wherein
said controller, after an interruption of a supply of power to said plurality of laser diodes, incrementally increases said supply of power at a rate slow enough that a nominal full power is not attainable until at least 200 milliseconds have elapsed since a reestablishment of said supply of power.

### Embodiment 26:

The laser irradiation device of Embodiment 25, wherein:
said controller incrementally increases said supply of power at a rate fast enough that said nominal full power is attainable within 5 seconds of said reestablishment of power.

### Embodiment 27:

A method, comprising:
interrupting a supply of power to a plurality of laser diodes, and
incrementally increasing said supply of power at a rate slow enough that a nominal full power is not attainable until at least 200 milliseconds have elapsed since a reestablishment of said supply of power.

### Embodiment 28:

The method of Embodiment 27, wherein:
said incrementally increasing said supply of power comprises incrementally increasing said supply of power at a rate fast enough that said nominal full power is attainable within 5 seconds of said reestablishment of power.

## Claims

1. **A laser irradiation device, comprising:**
a planar surface at least 10 cm times 10 cm in area,
a plurality of semiconductor light emitters; and
a controller, wherein
said plurality of semiconductor light emitters are distributed on said planar surface, and
said controller, after an interruption of a supply of power to said plurality of semiconductor light emitters, incrementally increases said supply of power at a rate slow enough that a nominal full power is not attainable until at least 200 milliseconds have elapsed since a reestablishment of said supply of power.

2. **An irradiation device, comprising:**
a first plurality of semiconductor light emitters;
a magnetic position sensor; and
a support, wherein
said support comprises a first planar surface,
said first plurality of semiconductor light emitters are distributed on said first planar surface,
each semiconductor light emitter of said first plurality of semiconductor light emitters emits light at a wavelength greater than 800 nm and less than 1100 nm, and
said irradiation device is structured to control operation of at least one semiconductor light emitter of said first plurality of semiconductor light emitters as a function of an output of said magnetic position sensor.

3. **An irradiation device, comprising:**
a first plurality of semiconductor light emitters;
a clock; and
a support, wherein
said support comprises a first planar surface,
said first plurality of semiconductor light emitters are distributed on said first planar surface,
each semiconductor light emitter of said first plurality of semiconductor light emitters emits light at a wavelength greater than 800 nm and less than 1100 nm, and
said irradiation device is structured to control operation of at least one semiconductor light emitter of said first plurality of semiconductor light emitters as a function of at least one of a current date and a current time established by said clock.

4. **An irradiation device, comprising:**
a first plurality of semiconductor light emitters;
a detector structured to distinguish between a presence and an absence of an irradiation target; and
a support, wherein
said support comprises a first planar surface,
said first plurality of semiconductor light emitters are distributed on said first planar surface,
each semiconductor light emitter of said first plurality of semiconductor light emitters emits light at a wavelength greater than 800 nm and less than 1100 nm,
said irradiation device is structured to effect a timing sequence in response to a signal from said detector indicative of a presence of an irradiation target, and
said timing sequence comprises controlling operation of at least one semiconductor light emitter of said first plurality of semiconductor light emitters as a function of time.

5. The irradiation device of claim 4, wherein:
said timing sequence comprises interrupting a supply of power to said first plurality of semiconductor light emitters for a first length of time measured from receipt of said signal.

6. The irradiation device of any one of claims 1 and 3-5, comprising:
a magnetic position sensor, wherein
said irradiation device is structured to control operation of at least one semiconductor light emitter of said first plurality of semiconductor light emitters as a function of an output of said magnetic position sensor.

7. The irradiation device of any one of claims 1, 2, 4, and 5, comprising:
a clock, wherein
said irradiation device is structured to control operation of at least one semiconductor light emitter of said first plurality of semiconductor light emitters as a function of a current date established by said clock.

8. The irradiation device of any one of claims 1-3, comprising:
a detector structured to distinguish between a presence and an absence of an irradiation target, wherein
said irradiation device is structured to control operation of at least one semiconductor light emitter of said first plurality of semiconductor light emitters as a function of time in response to a signal from said detector indicative of a presence of an irradiation target.

9. The irradiation device of any one of claims 1-3, comprising:
a detector structured to distinguish between a presence and an absence of an irradiation target, wherein
said irradiation device is structured to interrupt a supply of power to at least one semiconductor light emitter of said first plurality of semiconductor light emitters as a function of time in response to a signal from said detector indicative of a presence of an irradiation target.

10. The irradiation device of any one of the preceding claims, comprising:
a controller; and
an udder detector, wherein
said first planar surface is at least 30 cm times 20 cm in area, and
said controller uses signals obtained from said udder detector to interrupt a supply of power to at least one subset of said first plurality of semiconductor light emitters such that at least 80% of a light emitted by said first plurality of semiconductor light emitters irradiate an udder.

11. The irradiation device of any one of claims 2-9, comprising:
a second plurality of semiconductor light emitters, wherein
said support comprises a second planar surface,
said second plurality of semiconductor light emitters are distributed on said second planar surface,
each semiconductor light emitter of said second plurality of semiconductor light emitters emits light at a wavelength greater than 800 nm and less than 1100 nm,
each semiconductor light emitter of said first plurality of semiconductor light emitters emits light in a first direction, and
each semiconductor light emitter of said second plurality of semiconductor light emitters emits light in a second direction not more than 30° from opposite said first direction.

12. **A milking system, comprising:**
a carousel; and
an irradiation device in accordance with any one of the preceding claims, wherein:
said irradiation device is mounted to one of a lateral side and a rear side of a milking stall of said carousel.

13. **A milking system, comprising:**
a carousel;
a first irradiation device in accordance with any one of the preceding claims;
a second irradiation device in accordance with any one of the preceding claims, wherein:
said first irradiation device is mounted to an outer perimeter fence ambient to said carousel, and
said second irradiation device is mounted to said outer perimeter fence ambient to said carousel.

14. **A milking system, comprising:**
a carousel; and
an irradiation device, wherein:
said milking system is structured to interrupt a supply of power to said irradiation device as a function of an angular position of said carousel.

15. **A method, comprising:**
equipping a milking system with an irradiation device in accordance with any one of the preceding claims.

16. The method of claim 15, wherein:
said milking system comprises a carousel, and
said equipping comprises mounting said irradiation device to one of a lateral side of a milking stall of said carousel, a rear side of a milking stall of said carousel, and an outer perimeter fence ambient to said carousel.

17. **A method, comprising:**
switching an irradiation device mounted on a milking carousel to a light-emitting state at a first location during rotation of said milking carousel, and
switching said irradiation device to a non-light-emitting state at a second location during rotation of said milking carousel.

18. **A method, comprising:**
interrupting a supply of power to a plurality of semiconductor light emitters, and
incrementally increasing said supply of power at a rate slow enough that a nominal full power is not attainable until at least 200 milliseconds have elapsed since a reestablishment of said supply of power, wherein
said plurality of semiconductor light emitters are distributed on said planar surface of at least 10 cm times 10 cm in area.
